# EUROPEAN PATENT APPLICATION

(11) **EP 2 354 226 A1**
(43) Date of publication of application: **10.08.2011**
(21) Application number: 09833450.1
(22) Date of filing: 16.12.2009
(51) Int. Cl.: C12N 15/09, C12Q 1/68

(54) **METHOD FOR DETECTING CONTROLS FOR NUCLEIC ACID AMPLIFICATION, AND USE THEREOF**

(30) Priority: 16.12.2008 JP 2008320168
(71) Applicant: Arkray, Inc., Minami-ku Kyoto-shi Kyoto 601-8045 (JP)
(72) Inventor: HIRAI, Mitsuharu, Kyoto-shi, Kyoto 601-8045 (JP); MAJIMA, Satoshi, Kyoto-shi, Kyoto 601/8045 (JP); NAKAJIMA, Shinya, Kyoto-shi, Kyoto 601-8045 (JP); KAMATA, Tatsuo, Kyoto-shi, Kyoto 601-8045 (JP)
(74) Representative: Jones, Elizabeth Louise
(86) International application number: PCT/JP2009/070967
(87) International publication number: WO 2010/071147

(57) **Abstract**

The present invention provides a control detection method for easily detecting a positive control and a negative control simultaneously in one reaction system. An amplification reaction is carried out by adding a control template nucleic acid to a reaction system for detecting controls. The template nucleic acid can be amplified by a primer capable of amplifying an objective target sequence. An amplification region of the control template nucleic acid amplified by the primer can be hybridized with a detection probe capable of hybridizing to the target sequence. A Tm value (Tm_{C}) of a double-stranded nucleic acid composed of the amplification region of the control template nucleic acid amplified by the primer and the detection probe is set different from a Tm value (Tm_{A}) of a double-stranded nucleic acid composed of the target sequence and the detection probe. Thereby, it can be determined whether or not amplification occurs in a reaction system on the basis of the presence or absence of a peak at the Tm_{C} and it can be determined whether or not a reaction system is contaminated with undesired nucleic acid on the basis of the presence or absence of a peak at a temperature other than Tm_{C}.

SEQUENCE LISTING

<110>  ARKLAY INC.

<120>  Method for detecting controls for nucleic acid amplification, and use
thereof

<130>  42.13.105799

<140>  09833450.1
<141>  2009-12-16

<150>  JP 2008-320168
<151>  2008-12-16

<160>  45

<170>  PatentIn version 3.1

<210>  1
<211>  149
<212>  DNA
<213>  Artificial Sequence

<220>
<223>  pT7 insert

<400>  1
tcacctctgc ctaatcatct cattttcatg tcctatgttc aatcttccaa gctgtgcctt     60

gggtggcttt gttgcattga cattgacccg tataaagaat ttggagcttc tgtggagtta    120

ctctcttttt tgccttctga cttctttcc                                      149


<210>  2
<211>  20
<212>  DNA
<213>  Artificial Sequence

<220>
<223>  Forward Primer 1

<400>  2
cataagagga ctcttggact                                                 20


<210>  3
<211>  19
<212>  DNA
<213>  Artificial Sequence

<220>
<223>  Reverse primer 3

<400>  3
atttatgcct acagcctcc                                                  19


<210>  4
<211>  21
<212>  DNA
<213>  Artificial Sequence

<220>
<223>  Forward primer 3

<400>  4

tcacctctgc ctaatcatct c                                                21

<210>  5
<211>  20
<212>  DNA
<213>  Artificial Sequence

<220>
<223>  Reverse primer 2

<400>  5
ggaaagaagt cagaaggcaa                                                  20

<210>  6
<211>  22
<212>  DNA
<213>  Artificial Sequence

<220>
<223>  Probe 1

<400>  6
atgtccatgc cccaaagcca cc                                               22

<210>  7
<211>  22
<212>  DNA
<213>  Artificial Sequence

<220>
<223>  Probe 2

<400>  7
atgtccatgt cctaaagcca cc                                               22

<210>  8
<211>  22
<212>  DNA
<213>  Artificial Sequence

<220>
<223>  Probe 3

<400>  8
atcattaacc taatctcctc cc                                               22

<210>  9
<211>  33
<212>  DNA
<213>  Artificial Sequence

<220>
<223>  Inhibitory oligonucleotide

<400>  9
aggcttgaac agtaggacat gaacaagaga tga                                   33

<210>  10
<211>  22
<212>  DNA
<213>  Human papillomavirus

<400>  10
ggtggctttg gggcatggac at                                                22

<210>  11
<211>  40
<212>  DNA
<213>  Artificial Sequence

<220>
<223>  primer

<400>  11
taaattattg ttttctctta gatatgcaat aattttccca                             40

<210>  12
<211>  23
<212>  DNA
<213>  Artificial Sequence

<220>
<223>  primer

<400>  12
cccgagggtt gttgatgtcc atc                                               23

<210>  13
<211>  17
<212>  DNA
<213>  Artificial Sequence

<220>
<223>  probe

<400>  13
tcccaggaac ccataac                                                      17

<210>  14
<211>  35
<212>  DNA
<213>  Artificial Sequence

<220>
<223>  primer

<400>  14
tgatggaaaa attgaatgaa aacatcagga ttgta                                  35

<210>  15
<211>  30
<212>  DNA
<213>  Artificial Sequence

<220>
<223>  primer

<400>  15
tcaaaaatgt acttcagggc ttggtcaata                                        30

<210>  16
<211>  17
<212>  DNA
<213>  Artificial Sequence

<220>
<223>  probe

<400>  16
ccctgaatcc aggtaag                                                      17


<210>  17
<211>  17
<212>  DNA
<213>  Artificial Sequence

<220>
<223>  oligonucleotide

<400>  17
gcatggcttc cattggg                                                      17


<210>  18
<211>  17
<212>  DNA
<213>  Artificial Sequence

<220>
<223>  probe

<400>  18
cccaatggaa gccatgc                                                      17


<210>  19
<211>  27
<212>  DNA
<213>  Artificial Sequence

<220>
<223>  primer

<400>  19
tgaaatagtt gtcctagcac ctgacgc                                           27


<210>  20
<211>  29
<212>  DNA
<213>  Artificial Sequence

<220>
<223>  primer

<400>  20
caaaagactc tttcacatcc tccctttgg                                         29


<210>  21
<211>  19
<212>  DNA
<213>  Artificial Sequence

<220>
<223>  probe

<400>  21
gagacagagc attttacac                                                    19

<210>  22
<211>  31
<212>  DNA
<213>  Artificial Sequence

<220>
<223>  primer

<400>  22
agctttttat agtcacgtga cacagtcaaa c                                    31


<210>  23
<211>  20
<212>  DNA
<213>  Artificial Sequence

<220>
<223>  primer

<400>  23
cgcctttgct cctgccagag                                                 20


<210>  24
<211>  29
<212>  DNA
<213>  Artificial Sequence

<220>
<223>  probe

<400>  24
ccatatatat atatatataa gtaggagag                                       29


<210>  25
<211>  17
<212>  DNA
<213>  Artificial Sequence

<220>
<223>  oligonucleotide

<400>  25
gcatggcttc cattggg                                                    17


<210>  26
<211>  17
<212>  DNA
<213>  Artificial Sequence

<220>
<223>  probe

<400>  26
cccaatggaa gccatgc                                                    17


<210>  27
<211>  315
<212>  DNA
<213>  Homo sapiens

<400>  27
caataaaaat ttccccatca agatatacaa tatattttat ttatatttat agttttaaat     60

tacaaccaga gcttggcata ttgtatctat acctttatta aatgctttta atttaataaa 120

ttattgtttt ctcttagata tgcaataatt ttcccactat cattgattat ttcccgggaa 180

cccataacaa attacttaaa aaccttgctt ttatggaaag tgatattttg gagaaagtaa 240

aagaacacca agaatcgatg gacatcaaca accctcggga ctttattgat tgcttctgat 300

caaaatggag aaggt 315

<210> 28
<211> 316
<212> DNA
<213> Homo sapiens

<400> 28
caataaaaat ttccccatca agatatacaa tatattttat ttatatttat agttttaaat 60

tacaaccaga gcttggcata ttgtatctat acctttatta aatgctttta atttaataaa 120

ttattgtttt ctcttagata tgcaataatt ttcccactat cattgattat ttcccaggaa 180

cccataacaa attacttaaa aaccttgctt ttatggaaag tgatattttg gagaaagtaa 240

aagaacacca agaatcgatg gacatcaaca accctcggga ctttattgat tgcttcctga 300

tcaaaatgga gaaggt 316

<210> 29
<211> 354
<212> DNA
<213> Homo sapiens

<400> 29
actttcatcc tgggctgtgc tccctgcaat gtgatctgct ccattatttt ccagaaacgt 60

ttcgattata aagatcagca atttcttaac ttgatggaaa aattgaatga aaacatcagg 120

attgtaagca ccccctggat ccaggtaagg ccaagttttt tgcttcctga gaaaccactt 180

acagtctttt tttctgggaa atccaaaatt ctatattgac caagccctga agtacatttt 240

tgaatactac agtcttgcct agacagccat ggggtgaata tctggaaaag atggcaaagt 300

tctttatttt atgcacagga aatgaatatc ccaatataga tcaggcttct aagc 354

<210> 30
<211> 354
<212> DNA
<213> Homo sapiens

<400> 30
actttcatcc tgggctgtgc tccctgcaat gtgatctgct ccattatttt ccagaaacgt 60

ttcgattata aagatcagca atttcttaac ttgatggaaa aattgaatga aaacatcagg 120

attgtaagca ccccctgaat ccaggtaagg ccaagttttt tgcttcctga gaaaccactt 180

acagtctttt tttctgggaa atccaaaatt ctatattgac caagccctga agtacatttt 240

tgaatactac agtcttgcct agacagccat ggggtgaata tctggaaaag atggcaaagt 300

tctttatttt atgcacagga aatgaatatc ccaatataga tcaggcttct aagc 354

<210>  31
<211>  220
<212>  DNA
<213>  Homo sapiens

<400>  31
aagctccacc ttctttatct ctgaaagtga actccctgct acctttgtgg actgacagct      60

ttttatagtc acgtgacaca gtcaaacatt aacttggtgt atcgattggt ttttgccata     120

tatatatata taagtaggag agggcgaacc tctggcagga gcaaaggcgc catggctgtg     180

gagtcccagg gcggacgccc acttgtcctg ggcctgctgc                           220


<210>  32
<211>  222
<212>  DNA
<213>  Homo sapiens

<400>  32
aagctccacc ttctttatct ctgaaagtga actccctgct acctttgtgg actgacagct      60

ttttatagtc acgtgacaca gtcaaacatt aacttggtgt atcgattggt ttttgccata     120

tatatatata tataagtagg agagggcgaa cctctggcag gagcaaaggc gccatggctg     180

tggagtccca gggcggacgc ccacttgtcc tgggcctgct gc                        222


<210>  33
<211>  220
<212>  DNA
<213>  Homo sapiens

<400>  33
cagccactgg ctgagcatgc ttggggccat ccagcagctg cagcagaggg gacatgaaat      60

agttgtccta gcacctgacg cctcgttgta catcagagac ggagcatttt acaccttgaa     120

gacgtaccct gtgccattcc aaagggagga tgtgaaagag tcttttgtta gtctcgggca     180

taatgttttt gagaatgatt ctttcctgca gcgtgtgatc                           220


<210>  34
<211>  220
<212>  DNA
<213>  Homo sapiens

<400>  34
cagccactgg ctgagcatgc ttggggccat ccagcagctg cagcagaggg gacatgaaat      60

agttgtccta gcacctgacg cctcgttgta catcagagac agagcatttt acaccttgaa     120

gacgtaccct gtgccattcc aaagggagga tgtgaaagag tcttttgtta gtctcgggca     180

taatgttttt gagaatgatt ctttcctgca gcgtgtgatc                           220


<210>  35
<211>  300
<212>  DNA
<213>  Homo sapiens

<400>  35
ttcccatgat gatctgtccc tcacagcagg gtcttctctg tttcagggca tgaactactt      60

ggaggaccgt cgcttggtgc accgcgacct ggcagccagg aacgtactgg tgaaaacacc 120

gcagcatgtc aagatcacag attttgggct ggccaaactg ctgggtgcgg aagagaaaga 180

ataccatgca gaaggaggca aagtaaggag gtggctttag gtcagccagc attttcctga 240

caccagggac caggctgcct tcccactagc tgtattgttt aacacatgca ggggaggatg 300

<210> 36
<211> 300
<212> DNA
<213> Homo sapiens

<400> 36
ttcccatgat gatctgtccc tcacagcagg gtcttctctg tttcagggca tgaactactt 60

ggaggaccgt cgcttggtgc accgcgacct ggcagccagg aacgtactgg tgaaaacacc 120

gcagcatgtc aagatcacag attttgggcg ggccaaactg ctgggtgcgg aagagaaaga 180

ataccatgca gaaggaggca aagtaaggag gtggctttag gtcagccagc attttcctga 240

caccagggac caggctgcct tcccactagc tgtattgttt aacacatgca ggggaggatg 300

<210> 37
<211> 17
<212> DNA
<213> Artificial Sequence

<220>
<223> probe

<400> 37
ttggcccgcc caaaatc 17

<210> 38
<211> 25
<212> DNA
<213> Artificial Sequence

<220>
<223> primer

<400> 38
aggaacgtac tggtgaaaac accgc 25

<210> 39
<211> 28
<212> DNA
<213> Artificial Sequence

<220>
<223> primer

<400> 39
ttactttgcc tccttctgca tggtattc 28

<210> 40
<211> 300
<212> DNA
<213> Homo sapiens

<400> 40

tgaaataatt tttcatataa aggtgagttt gtattaaaag gtactggtgg agtatttgat 60

agtgtattaa ccttatgtgt gacatgttct aatatagtca cagtttcatt atttttatta 120

taaggcctgc tgaaaatgag tgaatataaa cttgtggtgc gtagccagca agttggagct 180

ggtggcgtag gcaagagtgc cttgacgata cagctaattc agaatcattt tgtggacgaa 240

tatgatccaa caatagaggt aaatcttgtt ttaatatgca tattactggt gcaggaccat 300

<210> 41
<211> 27
<212> DNA
<213> Artificial Sequence

<220>
<223> probe

<400> 41
ctcttgccta cgccaccagc tccaact 27

<210> 42
<211> 21
<212> DNA
<213> Artificial Sequence

<220>
<223> primer

<400> 42
aaggcctgct gaaaatgact g 21

<210> 43
<211> 22
<212> DNA
<213> Artificial Sequence

<220>
<223> primer

<400> 43
ggtcctgcac cagtaatatg ca 22

<210> 44
<211> 300
<212> DNA
<213> Homo sapiens

<400> 44
tgaaataatt tttcatataa aggtgagttt gtattaaaag gtactggtgg agtatttgat 60

agtgtattaa ccttatgtgt gacatgttct aatatagtca cagtttcatt atttttatta 120

taaggcctgc tgaaaatgag tgaatataaa cttgtggtgc gtagccagca agttggagct 180

gatggcgtag gcaagagtgc cttgacgata cagctaattc agaatcattt tgtggacgaa 240

tatgatccaa caatagaggt aaatcttgtt ttaatatgca tattactggt gcaggaccat 300

<210> 45
<211> 22
<212> DNA
<213> Artificial Sequence

<220>
<223>  oligonucleotide

<400>  45
ggtggctttg ttgcattgac at                                                22

## Description

### Technical Field

The present invention relates to a method for detecting controls showing amplification efficiency of a reaction system when a target sequence of a specimen nucleic acid is amplified. Specifically, the present invention relates to a method for amplifying a nucleic acid and a method for analyzing a melting curve each using the method for detecting controls, and a control detection reagent used therefor.

### Background Art

In current gene analysis, a method for analyzing a melting temperature (Tm) of a double-stranded nucleic acid composed of a target sequence and a probe is used widely. This method is called as a Tm analysis method or a melting curve analysis method since the method is conducted by analyzing a melting curve of the double-stranded nucleic acid, for example. According to this method, for example, the presence or absence of amplification of the target sequence can be determined and an objective polymorphism in the target sequence can be detected.

The presence or absence of amplification can be determined, for example, as follows. First, in the presence of a probe capable of hybridizing to the target sequence, the target sequence in a specimen nucleic acid is amplified and then the thus obtained amplification product of the target sequence is hybridized with the probe. Then, the thus obtained hybridization product is heat-treated, dissociation (melting) of the hybridization product accompanying the temperature rise is detected as signal values such as an absorbance and the like, and a Tm value (measured value) that shows the great variation in the signal value is determined. On the other hand, a Tm value (reference value for assessment) is determined beforehand with respect to a hybridization product between the target sequence and the probe. When the measured value is comparable to the reference value, it can be determined that the target sequence is amplified. When the Tm value cannot be obtained, it can be determined that the target sequence is not amplified. Further, a polymorphism can be detected, for example, as follows. First, the target sequence in a specimen nucleic acid is amplified in the presence of a probe capable of hybridizing to a target sequence whose objective target site is a mutant-type, and a Tm value is determined by detecting variations in a signal in the same manner as described above. The Tm value becomes higher as the complementarity of a hybridization product becomes higher and becomes lower as the complementarity of a hybridization product becomes lower. Therefore, a Tm value (reference value for assessment) is determined beforehand with respect to a hybridization product between the target sequence whose target site is a mutant-type and the probe, and this reference value is compared to the aforementioned measured Tm value. When the measured value is comparable to the reference value, it is considered as matching, that is, it can be determined that the target site in a specimen nucleic acid is a mutant-type. On the other hand, when the measured value is lower than the reference value, it is considered as mismatching, that is, it can be determined that the target site in a specimen nucleic acid is a wild-type.

When a gene analysis is performed using such a melting curve analysis, in addition to the detection of amplification of a specimen nucleic acid, the detection of a positive control and a negative control is carried out (see Patent Document 1). The positive control generally is a control that shows whether or not an amplification reaction occurs in a reaction system. The negative control is a control that shows whether or not a reaction system is contaminated with undesired nucleic acid. The positive control is needed for the following reasons. In nucleic acid amplification, when amplification of a target sequence is not detected, it is unknown whether the target sequence is not detected because the target sequence is not present in the specimen nucleic acid or the target sequence is not detected because an amplification reaction itself does not occur in a reaction system. Therefore, in addition to a reaction system for amplifying a specimen nucleic acid (hereinafter, referred to as a specimen reaction system), using a reaction system for a positive control (hereinafter, referred to as a positive control reaction system), nucleic acid amplification and Tm value analysis are performed in the same manner as described above. Conditions of the positive control reaction system are the same as those of the specimen reaction system except that a specimen nucleic acid is not added but a template nucleic acid containing the target sequence (a template nucleic acid for a positive control, hereinafter, referred to as a positive control template nucleic acid) is added. When the Tm value comparable to the reference value is obtained in the positive control reaction system, it is considered as "positive control (+)", that is, it can be determined that the amplification reaction occurs therein. Therefore, when the amplification in the specimen reaction system is not detected, it can be determined that the target sequence is not present in the specimen nucleic acid. Further, when the Tm value is not obtained in the positive control reaction system, it is considered as "positive control (-)", that is, it can be determined that the amplification reaction itself does not occur therein. The negative control is needed for the following reasons. There is a case where a reaction system is contaminated with a nucleic acid other than a specimen nucleic acid because the reaction system is contaminated with a cell or the like of an experimenter and amplification occurs from the undesired contaminating nucleic acid. In such a case, even when the variation in a signal is observed at the reference Tm value, it is unknown whether the amplification is derived from the specimen nucleic acid or the amplification is derived from the contaminating nucleic acid. Therefore, besides the specimen reaction system and the positive control reaction system, using a reaction system for a negative control (hereinafter, referred to as a negative control reaction system), nucleic acid amplification and Tm value analysis are performed in the same manner as described above. Conditions of the negative control reaction system are the same as those of the specimen reaction system except that a specimen nucleic acid is not added. When the Tm value is not obtained in the negative control reaction system, it is considered as "negative control (-)", that is, it can be determined that the reaction system is not contaminated with undesired nucleic acid. When the Tm value is obtained, it is considered as "negative control (+), that is, it can be determined that the amplification reaction occurrs because the reaction system is contaminated with undesired nucleic acid, and the analysis would be performed again.

In this manner, when an analysis of a specimen nucleic acid is performed, besides a specimen reaction system, a positive control reaction system and a negative control reaction system are required. However, an analysis device or the like has a limited number of measurement units. For example, in the case where the analysis device has four measurement units, the number of reaction systems that can be simultaneously analyzed is four. Therefore, in one analysis, half of the reaction systems is used for controls. Further, the positive control reaction system and the negative control reaction system are required for every analysis. Moreover, even in the case of analyzing only one specimen, two control reaction systems are required. When the number of control reaction systems is increased, costs for a reagent and the like are increased.

### Prior Art Document

### Patent Document

Patent Document 1: JP3331178 B

### Disclosure of the Invention

### Problem to be Solved by the Invention

Hence, the present invention is intended to provide a method for detecting controls that easily achieves, in one reaction system, simultaneous detection of a positive control showing an occurrence of an amplification reaction and a negative control showing an occurrence of contamination of a reaction system with undesired nucleic acid.

### Means for Solving Problem

In order to achieve the aforementioned object, the control detection method of the present invention is a method for detecting controls showing amplification efficiency of a reaction system when a target sequence of a specimen nucleic acid is amplified in the reaction system,
the controls being a positive control showing an occurrence of an amplification reaction in the reaction system and a negative control showing an occurrence of contamination of the reaction system with undesired nucleic acid,
the reaction system containing a primer, a detection probe, and a control template nucleic acid,
the primer being a primer capable of amplifying the target sequence,
the detection probe being a probe capable of hybridizing to the target sequence,
the primer being capable of amplifying the control template nucleic acid,
the detection probe being capable of hybridizing to an amplification region of the control template nucleic acid amplified by the primer,
a Tm value (Tm_{C}) of a double-stranded nucleic acid composed of the amplification region of the control template nucleic acid and the detection probe being different from a Tm value (Tm_{A}) of a double-stranded nucleic acid composed of the target sequence and the detection probe, the method including the following steps (a1) to (a3):
(a1) amplifying the control template nucleic acid in the reaction system using the primer;
(a2) performing a melting curve analysis in the presence of the detection probe using the reaction system obtained in the step (a1); and
(a3) determining whether the positive control and the negative control each are positive or negative on the basis of a peak in the melting curve, wherein the positive control and the negative control are detected in one reaction system.

The nucleic acid amplification method of the present invention is a method for amplifying a target sequence of a specimen nucleic acid in a reaction system, including the following steps (A) and (B):
(A) detecting controls showing amplification efficiency with respect to a reaction system containing the control template nucleic acid, the primer, and the detection probe by the control detection method of the present invention; and
(B) amplifying the target sequence of the specimen nucleic acid, including the following step (b1):
   (b1) amplifying the target sequence of the specimen nucleic acid in a reaction system containing the specimen nucleic acid, the primer, and the detection probe using the primer.

The melting curve analysis method of the present invention is a method for analyzing a melting curve of a reaction system containing a specimen nucleic acid, including the following steps (A) and (C):
(A) detecting controls showing amplification efficiency with respect to a reaction system containing the control template nucleic acid, the primer, and the detection probe by the control detection method of the present invention; and
(C) performing a melting curve analysis, including the following steps (c1) and (c2):
   (c1) amplifying a target sequence of the specimen nucleic acid in a reaction system containing the specimen nucleic acid, the primer, and the detection probe using the primer; and
   (c2) performing the melting curve analysis in the presence of the detection probe using the reaction system obtained in the step (c1).

The reagent of the present invention is a control detection reagent used for the control detection method of the present invention, including:
a control template nucleic acid, wherein
the control template nucleic acid can be amplified by a primer capable of amplifying an objective target sequence,
an amplification region of the control template nucleic acid amplified by the primer can be hybridized with a detection probe capable of hybridizing to the target sequence, and
a Tm value (Tm_{C}) of a double-stranded nucleic acid composed of the amplification region of the control template nucleic acid and the detection probe is different from a Tm value (Tm_{A}) of a double-stranded nucleic acid composed of the target sequence and the detection probe.

### Effects of the Invention

According to the present invention, a positive control and a negative control, which had to be detected separately, can be detected in one reaction system using the aforementioned control template nucleic acid. In this manner, by detecting the positive control and the negative control in one reaction system, for example, operations can be simplified and reagent costs and the like can be reduced.

### Brief Description of Drawings

[FIG. 1] FIG. 1 is a schematic view showing hybridization between a probe and a target sequence and hybridization between a probe and a control template nucleic acid in an embodiment of the present invention.
[FIG. 2] FIGs. 2A to 2C are schematic views showing melting curves in another embodiment of the present invention.
[FIG. 3] FIG. 3 is a schematic view showing hybridization between a probe and a target sequence and hybridization between a probe and a control template nucleic acid in another embodiment of the present invention.
[FIG. 4] FIG. 4 is a schematic view showing hybridization between a probe and a target sequence and hybridization between a probe and a control template nucleic acid in yet another embodiment of the present invention.
[FIG. 5] FIG. 5 is a schematic view showing hybridization between a probe and a target sequence and hybridization between a probe and a control template nucleic acid in still another embodiment of the present invention.
[FIG. 6] FIGs. 6A and 6B show graphs indicating results of the Tm analysis in Example A1 of the present invention.
[FIG. 7] FIGs. 7A and 7B show graphs indicating results of the Tm analysis in Example A2 of the present invention.
[FIG. 8] FIGs. 8A and 8B show graphs indicating results of the Tm analysis in Example A2 of the present invention.
[FIG. 9] FIGs. 9A and 9B show graphs indicating results of the Tm analysis in Example A3 of the present invention.
[FIG. 10] FIGs. 10A and 10B show graphs indicating results of the Tm analysis in Example A3 of the present invention.
[FIG. 11] FIGs. 11A and 11B show graphs indicating results of the Tm analysis in Example A4 of the present invention.
[FIG. 12] FIGs. 12A and 12B show graphs indicating results of the Tm analysis in Example A4 of the present invention.
[FIG. 13] FIGs. 13A and 13B show graphs indicating results of the Tm analysis in Example B1 of the present invention.
[FIG. 14] FIGs. 14A and 14B show graphs indicating results of the Tm analysis in Example B1 of the present invention.
[FIG. 15] FIG. 15 shows graphs indicating results of the Tm analysis in Example B1 of the present invention.
[FIG. 16] FIGs. 16A and 16B show graphs indicating results of the Tm analysis in Example B2 of the present invention.
[FIG. 17] FIGs. 17A and 17B show graphs indicating results of the Tm analysis in Example B2 of the present invention.
[FIG. 18] FIG. 18 shows graphs indicating results of the Tm analysis in Example B2 of the present invention.

### Best Mode for Carrying out the Invention

In the present invention, a "target sequence" is an objective region to be amplified in a specimen nucleic acid, i.e., a sequence that is amplified by the primer. In the present invention, a "target site" is a base site in which an objective polymorphism occurs in the target sequence of the specimen nucleic acid. When the polymorphism is a mutant-type, the target site is also referred to as a "mutant-type target site" and when the polymorphism is a wild-type, the target site is also referred to as a "wild-type target site". The wild-type can be referred to as a normal-type. A target sequence having a wild-type target site is also referred to as a "wild-type target sequence" and a target sequence having a mutant-type target site is also referred to as a "mutant-type target sequence". A region of the control template nucleic acid amplified by a primer is also referred to as an "amplification region of the control template nucleic acid". In the target sequence of the specimen nucleic acid and in the amplification region of the control template nucleic acid, regions to which the detection probe can hybridize are each referred to as a "detection probe-hybridizing region", hereinafter. In the present invention, the hybridization region is a region forming a double-stranded nucleic acid as a whole. The hybridization region may be a region in which whole bases in the region form base pairing by a hydrogen bond or a region containing mismatching bases that do not form base pairing in the region, for example. The mismatching may be a state in which corresponding bases are not bound by a hydrogen bond such as a combination of adenine and cytosine or a state in which bases at corresponding base sites between the sequences are deleted. Further, in the present invention, a region excluding the hybridization region is also referred to as a "non-hybridization region". In the present invention, a reaction system for amplifying a control template nucleic acid is also referred to as a "control reaction system" and a reaction system for amplifying a target sequence of a specimen nucleic acid is also referred to as a "specimen reaction system". In the present invention, hereinafter, a "control template nucleic acid" for detecting a positive control and a negative control is also referred to as a "control template".

In the present invention, a "positive control" is also referred to as an "amplification control" because it shows occurrence of an amplification reaction in a reaction system. Positive control (+) indicates that an amplification reaction occurs. Positive control (-) indicates that an amplification reaction does not occur. In the present invention, a "negative control" is also referred to as a "contamination control" because it shows production of an amplification product of the undesired nucleic acid because of contamination of a reaction system with undesired nucleic acid. Negative control (+) indicates that an amplification product of the undesired nucleic acid is produced because the reaction system is contaminated with undesired nucleic acid. Negative control (-) indicates that a reaction system is not contaminated with undesired nucleic acid or an amplification product from the undesired nucleic acid is not produced even when the reaction system is contaminated with undesired nucleic acid.

### <Control Detection Method>

As described above, the control detection method of the present invention is a method for detecting controls showing amplification efficiency of a reaction system when a target sequence of a specimen nucleic acid is amplified in the reaction system,
the controls being a positive control showing an occurrence of an amplification reaction in the reaction system and a negative control showing an occurrence of contamination of the reaction system with undesired nucleic acid,
the reaction system containing a primer, a detection probe, and a control template nucleic acid,
the primer being a primer capable of amplifying the target sequence,
the detection probe being a probe capable of hybridizing to the target sequence,
the control template nucleic acid capable of being amplified by the primer,
an amplification region of the control template nucleic acid amplified by the primer capable of being hybridized with the detection probe,
a Tm value (Tm_{C}) of a double-stranded nucleic acid composed of the amplification region of the control template nucleic acid and the detection probe being different from a Tm value (Tm_{A}) of a double-stranded nucleic acid composed of the target sequence and the detection probe,
the method including the following steps (a1) to (a3):
(a1) amplifying the control template nucleic acid in the reaction system using the primer;
(a2) performing a melting curve analysis in the presence of the detection probe using the reaction system obtained in the step (a1); and
(a3) judging whether the positive control and the negative control each are positive or negative on the basis of a peak in the melting curve, wherein the positive control and the negative control are detected in one reaction system.

According to the control detection method of the present invention, as described above, when amplification of an objective specimen nucleic acid is carried out, the determination of whether or not an amplification reaction occurs normally in a reaction system and the determination of whether or not amplification because of undesired nucleic acid occurs in a reaction system can be made using a control reaction system that is different from a reaction system for amplifying the specimen nucleic acid. As described above, the determination of amplification efficiency in a reaction system to be used is important for determining whether or not amplification occurs with high accuracy when an objective specimen nucleic acid is amplified. Therefore, in the control detection method of the present invention, the step (a1) of amplifying the control template is preferably performed, for example, in advance of, simultaneously, or after a step of amplifying the specimen nucleic acid using the specimen reaction system under the same conditions. Above all, for example, since the conditions such as time for amplification reaction, temperature, and the like for the step (a1) of amplifying the control template can be substantially the same as those for the step of amplifying the specimen nucleic acid, it is preferable that these steps are performed simultaneously. Specifically, it is preferable to simultaneously perform respective amplification reactions with respect to the control reaction system and the specimen reaction system using a device (for example, an amplification device) provided with a plurality of measurement units. This will be described in detail in the nucleic acid amplification method of the present invention.

The control reaction system in the step (a1) is a reaction system for detecting a positive control and a negative control. The control reaction system does not contain a specimen nucleic acid but contains a control template as an amplification template. The conditions of the control reaction system used for amplification of the control template are preferably the same as those of the specimen reaction system used for amplification of the specimen nucleic acid except that the former contains the control template and the latter contains the specimen nucleic acid. With respect to the control template and the specimen nucleic acid, the control reaction system contains the control template and does not contain the specimen nucleic acid whereas the specimen reaction system contains the specimen nucleic acid and may contain or may not contain the control template, for example.

The control template may be, for example, a single-stranded nucleic acid or a double-stranded nucleic acid. When the control template is a double-stranded nucleic acid, for example, although either one of two single-stranded nucleic acids of the double-stranded nucleic acid can be used as a template for nucleic acid amplification, from the viewpoint of amplification efficiency, it is preferable that both of them are used as templates. Either one of two single-stranded nucleic acids of the double-stranded nucleic acid can be a sense strand or an antisense strand.

In the control detection method of the present invention, the primer and the detection probe are the same as those used in a specimen reaction system for amplifying a specimen nucleic acid. Therefore, it is required that the control template can be amplified by a primer capable of amplifying a target sequence of the specimen nucleic acid and can be hybridized with a detection probe capable of hybridizing to a target sequence of the specimen nucleic acid. These can be achieved by designing the control template as a base sequence having, at the 3' side, a sequence complementary to the primer and having, at the 5' side relative to the aforementioned sequence, a sequence capable of hybridizing to the detection probe. The "sequence complementary to the primer" is applicable as long as the primer can start elongation by annealing, for example. The degree of complementarity is, for example, 50% or more, preferably 80% or more, and more preferably 100%, although it is not particularly limited. The "sequence capable of hybridizing to the detection probe" may be, for example, a sequence complementary to the whole detection probe or a sequence complementary to a part of the detection probe, and it can be set suitably according to, for example, the relationship with the Tm value that will be described later.

Further, it is required that the Tm value (Tm_{C}) of a double-stranded nucleic acid composed of the amplification region of the control template and the detection probe is different from the Tm value (Tm_{A}) of a double-stranded nucleic acid composed of the target sequence and the detection probe. This can be achieved by setting conditions of a detection probe-hybridizing region in the target sequence different from those of a detection probe-hybridizing region in the control template. Specifically, for example, this can be achieved by changing the lengths of the respective hybridization regions or by changing the homology between the respective hybridization regions. The sequence of the amplification region in the control template is preferably the same as that of the target sequence except for a part of the detection probe-hybridizing region.

First, the former method in which the lengths of the respective hybridization regions are changed will be described. For example, the Tm value becomes higher as the length of a hybridization region becomes relatively longer and becomes lower as the length of a hybridization region becomes relatively shorter. Therefore, when the same detection probe is used, for example, by setting the length of the hybridization region in the target sequence different from that of the hybridization region in the control template, the Tm values thereof can be set as different values. That is, in this embodiment, the control template is designed such that the length of the detection probe-hybridizing region in the control template is different from that of the detection probe-hybridizing region in the target sequence. In this case, for example, the length of the hybridization region in the target sequence may be longer than that of the hybridization region in the control template, or the length of the hybridization region in the control template may be longer than that of the hybridization region in the target sequence. The relationship between the hybridization region in the control template and the hybridization region in the target sequence will be described with specific examples.

First, as a first embodiment, the case in which the length of the hybridization region in the target sequence is longer than that of the hybridization region in the control template will be described. In this case, for example, it is preferable to use a probe consisting of a base sequence complementary to a predetermined region of the target sequence as the detection probe. Specifically, it is preferable to use a probe consisting of a base sequence 100% complementary thereto. Further, the control template preferably has, at the amplification region thereof, a base sequence to which the detection probe can partially hybridize. According to this embodiment, for example, the detection probe can wholly hybridize to the target sequence whereas the detection probe can partially hybridize to the control template. Therefore, the length of the hybridization region in the target sequence becomes longer than that of the hybridization region in the control template. With respect to the hybridization region in the target sequence and the hybridization region in the control template, a region to which the detection probe can hybridize is also referred to as a consensus region or a consensus base sequence.

The control template preferably has, for example, at the amplification region thereof, a base sequence in which at least one of the 5' side and the 3' side is different from that of a detection probe-hybridizing region in the target sequence. Alternatively, it can be said that the control template preferably has, for example, at the amplification region thereof, a base sequence complementary to a sequence in which at least one of the 5' region and the 3' region is different from that of the base sequence of the detection probe. By designing the control template in this manner, for example, one of the 5' region and the 3' region of the probe hybridizes to the control template and the other does not hybridize to the control template.

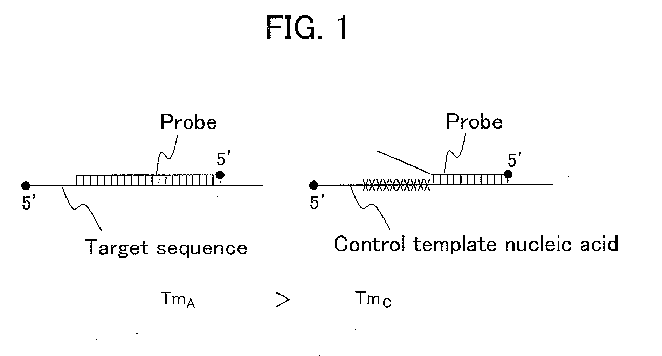

A specific example of the first embodiment will be described using FIG.1. The present invention is not limited thereto. In FIG. 1, the view on the left is a schematic view showing a state in which the detection probe hybridizes to the target sequence, and the view on the right is a schematic views showing a state in which the detection probe hybridizes to the amplification region of the control template. As shown in the view on the left, the whole detection probe hybridizes to a predetermined region of the target sequence. On the other hand, as shown in the view on the right, the control template has, at the amplification region thereof, a base sequence complementary to a sequence in which the 3' region is different from that of the base sequence of the detection probe. In other words, the control template in the view on the right has a base sequence in which the 5' region (indicated by "X" in the view on the right) is different from that of the detection probe-hybridizing region in the target sequence shown in the view on the left. Therefore, the 3' region of the detection probe does not hybridize to the control template. In other words, the 3' region of the detection probe is a non-hybridization region. In this manner, even when the same detection probe is used, the Tm value of a double-stranded nucleic acid in which the detection probe is wholly hybridized is higher than that of a double-stranded nucleic acid in which the detection probe is partially hybridized (Tm_{A} > Tm_{C}).

FIG. 1 shows an example in which the 3' region of the detection probe does not hybridize to the control template. The present invention is not limited thereto, and it is also possible that the 5' region of the detection probe does not hybridize to the control template.

The length of the detection probe is not particularly limited, and is, for example, 5 to 50 bases, and preferably 10 to 30 bases. In the detection probe, the length of the base sequence hybridizing to the control template is, for example, preferably 5 to 40 bases, more preferably 10 to 30 bases, and yet more preferably 15 to 25 bases. This length corresponds to, for example, the length of the base sequence to which the detection probe can partially hybridize in the amplification region of the control template, and it can be the length of the consensus sequence between the hybridization region in the control template and the hybridization region in the target sequence. Further, in the detection probe, the length of the base sequence of the non-hybridization region that does not hybridize to the control template is, for example, preferably 1 to 40 bases, more preferably 3 to 20 bases, and yet more preferably 5 to 10 bases. This length corresponds to, for example, in the amplification region of the control template, the length of the sequence of at least one of the 5' side and the 3' side that is different from the base sequence of the detection probe-hybridizing region in the target sequence, and it can be the length of the sequence (non-consensus sequence) of the hybridization region in the target sequence excluding the consensus sequence between the hybridization region in the control template and the hybridization region in the target sequence. In the view on the right of FIG. 1, for example, this length corresponds to the length of the non-hybridization region (indicated by "X" in the view on the right) to which the detection probe does not hybridize. Further, when the length of the hybridization region in the target sequence is assumed as 100%, the length of the hybridization region in the control template is, for example, preferably 50% to 95% thereof, and more preferably 75% to 90% thereof.

Next, as a second embodiment, the case in which the length of the hybridization region in the control template is longer than that of the hybridization region in the target sequence will be described. In this case, for example, a probe consisting of a base sequence partially complementary to the target sequence is used as the detection probe. In other words, as the detection probe, a probe having a hybridization region and a non-hybridization region with respect to the target sequence is used. The control template preferably has, for example, at the amplification region thereof, a base sequence that is identical to the detection probe-hybridizing region in the target sequence, and, adjacent thereto, a base sequence that is different from a region adjacent to at least one of the 5' side and the 3' side of the hybridization region in the target sequence. Specifically, the base sequence of the control template that is different from the region adjacent to at least one of the 5' side and the 3' side of the hybridization region in the target sequence is preferably adjacent to at least one of the 5' side and the 3' side of the base sequence of the control template that is identical to the hybridization region in the target sequence. More specifically, the base sequence of the control template that is different from the region adjacent to at least one of the 5' side and the 3' side of the hybridization region in the target sequence is preferably adjacent to at least one of the 5' end and the 3' end of the base sequence of the control template that is identical to the hybridization region in the target sequence. The region adjacent to at least one of the 5' side and the 3' side of the hybridization region in the target sequence is, for example, a region corresponding to the detection probe when the target sequence and the detection probe are in alignment, and is a region to which a part of the detection probe does not hybridize, that is, a non-hybridization region. According to this embodiment, for example, the detection probe can wholly hybridize to the control template whereas the detection probe can partially hybridize to the target sequence. Therefore, the length of the hybridization region in the control template becomes longer than that of the hybridization region in the target sequence. With respect to the hybridization region in the target sequence and the hybridization region in the control template, a region to which the detection probe can hybridize is also referred to as a consensus region or a consensus base sequence.

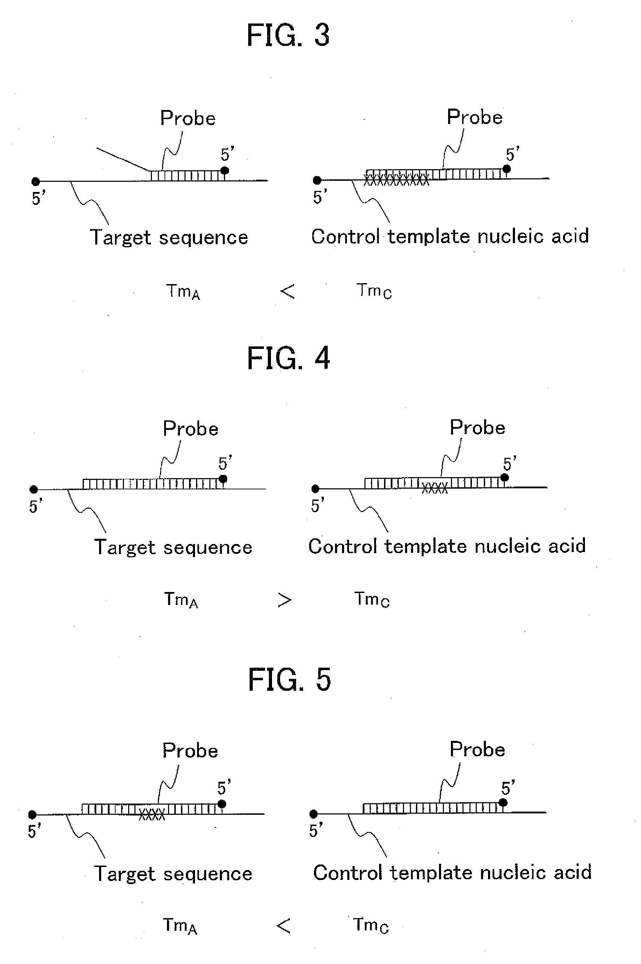

A specific example of the second embodiment will be described using FIG.3. The present invention is not limited thereto. In FIG. 3, the view on the left is a schematic view showing a state in which the detection probe hybridizes to the target sequence, and the view on the right is a schematic view showing a state in which the detection probe hybridizes to the control template. As shown in the view on the left, the detection probe is a base sequence complementary to a predetermined region of the target sequence excluding the 3' region thereof. Therefore, the 3' region of the detection probe does not hybridize to the target sequence. That is, the 3' region of the detection probe is a non-hybridization region. On the other hand, the control template in the view on the right has a base sequence in which the 5' region (indicated by "X" in the view on the right) is different from the non-hybridization region in the target sequence to which the detection probe does not hybridize shown in the view on the left. In other words, the control template has a base sequence complementary to the whole detection probe. Therefore, the whole detection probe hybridizes to the control template and the 3' region of the detection probe does not hybridize to the target sequence. In this manner, the Tm value of a double-stranded nucleic acid in which the detection probe is wholly hybridized is higher than that of a double-stranded nucleic acid in which the detection probe is partially hybridized (Tm_{C} >Tm_{A}) when the target sequence and the control template are hybridized with the same detection probe.

FIG. 3 shows an example in which the 3' region of the detection probe does not hybridize to the target sequence. The present invention is not limited thereto, and it is also possible that the 5' region of the detection probe does not hybridize to the target sequence.

As described above, the detection probe is preferably consisting of a base sequence partially complementary to the target sequence. In the detection probe, the length of the base sequence hybridizing to the target sequence is, for example, preferably 5 to 40 bases, more preferably 10 to 30 bases, and yet more preferably 15 to 25 bases. This length corresponds to, for example, in the hybridization region in the control template, the length of the base sequence that is in common with the base sequence of the hybridization region in the target sequence, and it can be the length of the consensus sequence between the hybridization region in the control template and the hybridization region in the target sequence. Further, in the detection probe, the length of the base sequence of the non-hybridization region that does not hybridize to the target sequence is, for example, preferably 1 to 40 bases, more preferably 3 to 20 bases, and yet more preferably 5 to 10 bases. This length corresponds to, for example, in the hybridization region in the control template, the length of the base sequence that is different from the base sequence of the hybridization region in the target sequence, and it can be the length of the sequence (non-consensus sequence) of the hybridization region in the control template excluding the consensus sequence between the hybridization region in the control template and the hybridization region in the target sequence. In the view on the right of FIG. 3, for example, this length corresponds to the length of a region (indicated by "X" in the view on the right) in which the detection probe hybridizes to the control template excluding the consensus sequence. Further, when the length of the hybridization region in the control template is assumed as 100%, the length of the hybridization region in the target sequence is, for example, preferably 50% to 95% thereof, and more preferably 75% to 90% thereof.

Next, the latter method in which the homology between the respective hybridization regions is changed will be described. For example, in a detection probe-hybridizing region, the Tm value becomes higher as the complementarity to the detection probe becomes relatively higher and becomes lower as the complementarity to the detection probe becomes relatively lower. Therefore, when the same detection probe is used, for example, the homology between the base sequence of the hybridization region in the control template and the base sequence of the hybridization region in the target sequence is set less than 100%. Thereby, the complementarity of the hybridization region in the control template to the detection probe can be set as a value that is different from the complementarity of the hybridization region in the target sequence to the detection probe, and thereby the Tm values can be set as different values. In this case, for example, the complementarity of the target sequence to the detection probe may be set higher than that of the control template to the detection probe, or the complementarity of the control template to the detection probe may be set higher than that of the target sequence to the detection probe. In this embodiment, for example, the hybridization region in the target sequence and the hybridization region in the control template may have the same length. The relationship between the hybridization region in the control template and the hybridization region in the target sequence will be described hereinafter with specific examples.

The homology between the hybridization regions is, for example, preferably less than 100%, more preferably less than 95%, and yet more preferably less than 90%. The number of bases that are different between the hybridization region in the control template and the hybridization region in the target sequence is not particularly limited, however is, for example, preferably 1 to 20 bases, more preferably 1 to 10 bases, and yet more preferably 1 to 5 bases. The bases that are different between the hybridization region in the control template and the hybridization region in the target sequence may be continuous or discontinuous in the hybridization regions. Further, with respect to the complementarity of the hybridization region in the control template to the detection probe and the complementarity of the hybridization region in the target sequence to the detection probe, for example, it is applicable as long as the complementarity of one of them is higher than the other. Specifically, the complementarity that is relatively higher, is, for example, 90 to 100%, and preferably 100%, and the complementarity that is relatively lower, is, for example, preferably 5 to 10% lower than the complementarity that is relatively higher.

As a first embodiment, the case in which the complementarity of the target sequence to the detection probe is higher than that of the control template to the detection probe is described using FIG. 4. The present invention is not limited thereto.

FIG. 4 shows an example in which a detection probe consisting of a base sequence 100% complementary to a predetermined region of the target sequence is used, and the base sequence of a hybridization region in the control template is set such that the complementarity to the detection probe is less than 100%. In FIG. 4, the view on the left is a schematic view showing a state in which the detection probe hybridizes to the target sequence, and the view on the right is a schematic view showing a state in which the detection probe hybridizes to an amplification region of the control template. As described above, the detection probe is a sequence 100% complementary to a predetermined region of the target sequence. Therefore, as shown in the view on the left, the whole detection probe hybridizes to a predetermined region of the target sequence and all of the bases form base pairing in the hybridization region. On the other hand, the control template is a sequence whose complementarity to the detection probe is less than 100%.
Specifically, the control template contains mismatching bases that are not complementary to the detection probe in the region indicated by "X" in the view on the right. In other words, the control template has a sequence whose homology is different from a predetermined region (hybridization region) in the target nucleic acid. Therefore, as shown in the view on the right, although the whole detection probe hybridizes to the control template, mismatching (indicated by "X" in the view on the right) in which base pairing is not formed occurs in the hybridization region. Thus, the complementarity of the control template to the detection probe becomes lower than that of the target sequence to the detection probe. Accordingly, even when the same detection probe is used, the Tm value of a double-stranded nucleic acid in which the whole hybridization region forms base pairing is higher than that of a double-stranded nucleic acid that contains mismatching in which the base pairing is not formed in the hybridization region (Tm_{A} > Tm_{C}). In FIG. 4, the probe is a sequence 100% complementary to a predetermined region of the target sequence. The present invention is not limited thereto and the probe may be a sequence that contains mismatching with the predetermined region. In this case, for example, when the amount of mismatching with the control template is larger than that of mismatching with the target sequence, the same result as described above can be obtained.

As a second embodiment, the case in which the complementarity of the control template to the detection probe is higher than that of the target sequence to the detection probe is described using FIG. 5. The present invention is not limited thereto.

FIG. 5 shows an example in which a detection probe consisting of a base sequence 100% complementary to a predetermined region of the control template is used, and a base sequence of a hybridization region in the target sequence is set such that the complementarity to the detection probe is less than 100%. In FIG. 5, the view on the left is a schematic view showing a state in which the detection probe hybridizes to the target sequence, and the view on the right is a schematic view showing a state in which the detection probe hybridizes to an amplification region of the control template. As described above, the detection probe is a sequence 100% complementary to a predetermined region of the control template. Therefore, as shown in the view on the right, the whole detection probe hybridizes to a predetermined region of the control template and all of the bases form base pairing in the hybridization region. On the other hand, the target sequence is a sequence whose complementarity to the detection probe is less than 100%.
Specifically, the control template contains mismatching bases not complementary to the detection probe in the region indicated by "X" in the view on the left. In other words, the target sequence has a sequence whose homology is different from a predetermined region (hybridization region) in the control template. Therefore, as shown in the view on the left, although the whole detection probe hybridizes to the target sequence, mismatching (indicated by "X" in the view on the right) in which base pairing is not formed occurs in the hybridization region. Thus, the complementarity of the target sequence to the detection probe becomes lower than that of the control template to the detection probe. Accordingly, even when the same detection probe is used, the Tm value of a double-stranded nucleic acid in which the whole hybridization region forms base pairing is higher than that of a double-stranded nucleic acid that contains mismatching in which the base pairing is not formed in the hybridization region (Tm_{C} > Tm_{A}). In FIG. 5, the probe is a sequence 100% complementary to a predetermined region of the control template. The present invention is not limited thereto and the probe may be a sequence that contains mismatching with the predetermined region. In this case, for example, when the amount of mismatching with the target sequence is larger than that of mismatching with the control template, the same result as described above can be obtained.

As a method for setting the Tm value (Tm_{C}) of a double-stranded nucleic acid composed of the amplification region of the control template nucleic acid and the detection probe different from the Tm value (Tm_{A}) of a double-stranded nucleic acid composed of the target sequence and the detection probe is not limited to the aforementioned method. For example, it is also possible to adjust the Tm value by using artificial nucleic acids such as LNA that is an RNA analog, PNA that is a peptide nucleic acid, BNA that is a bridged nucleic acid, and the like. Specifically, the Tm value can be adjusted by causing the artificial nucleic acid to be contained in the hybridization region in the control template, a site of the detection probe that hybridizes to only one of the target sequence and the control template, or the like..

The difference between the Tm value (Tm_{C}) of a double-stranded nucleic acid composed of the amplification region of the control template nucleic acid and the detection probe and the Tm value (Tm_{A}) of a double-stranded nucleic acid composed of the target sequence and the detection probe is not particularly limited. The difference is, for example, preferably in the range from 1 to 50°C, more preferably in the range from 3 to 30°C, and particularly preferably in the range from 5 to 50°C.

The length of the control template is not particularly limited, and can be decided suitably depending on, for example, the target sequence. The length of the control template is, for example, preferably 0 to 1,000,000 bases longer than the target sequence, more preferably 100 to 100,000 bases longer than the target sequence, and yet more preferably 1,000 to 10,000 bases longer than the target sequence. Specifically, the length of the control template is, for example, preferably 100 to 1,000,000 bases, more preferably 500 to 100,000 bases, and yet more preferably 1,000 to 10,000 bases. Further, the length of the amplification region of the control template is not particularly limited. The length of the amplification region of the control template is, for example, preferably 20 to 100,000 bases, more preferably 50 to 10,000 bases, and yet more preferably 100 to 1,000 bases.

In the present invention, the type of the primer is not particularly limited and a primer that amplifies only one of a sense strand and an antisense strand may be used. From the viewpoint of amplification efficiency, it is preferable to use two or more primers that amplify both the sense strand and the antisense strand.

The length of the primer is not particularly limited. Generally, the length is, for example, 10 to 50 bases, preferably 15 to 40 bases, and more preferably 16 to 35 bases.

In the present invention, the detection probe may be designed so as to hybridize to a sense strand or may be designed so as to hybridize to an antisense strand. In the present invention, "capable of hybridizing to a target sequence" may mean that the probe is capable of hybridizing to a target sequence or the probe is capable of hybridizing to a complementary strand of the target sequence.

The detection probe is applicable as long as it can hybridize to the target sequence and the detection probe can be designed to hybridize to any region of the target sequence. Further, as described later, the present invention can be utilized effectively also in detection of a polymorphism by a melting curve analysis. In this case, it is preferable to design the detection probe such that it can hybridize to a region containing a base site in which an objective polymorphism occurs in the target sequence. The detection of the polymorphism will be described later.

The length of the detection probe is not particularly limited. Generally, the length is, for example, 5 to 50 bases and preferably 10 to 30 bases.

The detection probe may be, for example, an unlabeled probe not having a labeling substance or a labeled probe having a labeling substance. With respect to the labeled probe, it is preferable that an oligonucleotide capable of hybridizing to the target sequence is labeled (modified) with the labeling substance. In the oligonucleotide, the site to be labeled with the labeling substance is not particularly limited. The site to be labeled with the labeling substance is, for example, preferably the 5' end region or the 3' end region and more preferably the 5' end or the 3' end. Further, as described later, in the oligonucleotide, the base to be labeled with the labeling substance is preferably cytosine (c) or guanine (g), for example. The base may be directly labeled with the labeling substance or the base may be indirectly labeled with the labeling substance through labeling of any site of a nucleotide residue containing the base. The labeling substance may be bound to either the 3' side or the 5' side of the detection probe. Further, for example, in order to prevent elongation of the probe itself in a nucleic acid amplification reaction, the detection probe further may include a phosphate group or the aforementioned labeling substance added to the 3' end thereof. [0047]
The labeling substance is not particularly limited, and is preferably the one that exhibits a signal depending on whether the labeled probe is present as itself or the labeled probe forms a hybridization product, for example. The type of the signal is not particularly limited, and examples thereof include fluorescence, coloring (color change or color development), and the like. In the case where the signal is fluorescence, an example of the signal value includes fluorescence intensity. In the case where the signal is coloring (color change or color development), examples of the signal values include a reflectance, an absorbance, a transmittance, and the like. Further, the signal may be emitted directly or indirectly from the labeling substance.

The labeling substance is not particularly limited, and examples thereof include fluorescent substances such as a fluorophore and the like. Examples of the fluorescent substances include fluorescein, phosphor, rhodamine, polymethine dye derivatives, and the like. Examples of the commercially available fluorescent substances include Pacific Blue™ (produced by Molecular Probe Inc.), BODIPY FL™ (produced by Molecular Probe Inc.), FluorePrime (product name, produced by Amersham Pharmacia), Fluoredite (product name, produced by Millipore Corporation), FAM™ (produced by ABI), Cy3 and Cy5 (product names, produced by Amersham Pharmacia), TAMRA™ (produced by Molecular Probe Inc.), and the like. Conditions for detecting the fluorescent substances are not particularly limited and can be decided suitably depending on the type of the fluorescent substance to be used. For example, Pacific Blue™ can be detected at the detection wavelength of 450 nm to 480 nm, TAMRA™ can be detected at the detection wavelength of 585 nm to 700 nm, and the BODIPY FL™ can be detected at the detection wavelength of 515 nm to 555 nm. By using such a probe, hybridization and disassociation can be recognized easily on the basis of the change in fluorescence intensity by detecting fluorescence as a signal and by measuring fluorescence intensity as a signal value.

Preferably, the labeled probe is, for example, a labeled probe that exhibits a signal independently but does not exhibit a signal by hybridization, or a labeled probe that does not exhibit a signal independently but exhibits a signal by hybridization. In the case where the labeling substance is a fluorescent substance, it is preferable that the labeled probe is, for example, a probe that is labeled with the fluorescent substance, exhibits fluorescence independently, and allows fluorescence to be reduced (for example, quenched) by hybridization. Such a phenomenon is generally called as a quenching phenomenon. A probe that utilizes this phenomenon is generally called as a fluorescence quenching probe. Above all, preferred is the probe labeled with the fluorescent substance at the 3' end of an oligonucleotide or the 5' end of the same, and the end base that is labeled is preferred to be cytosine (c) or guanine (g). In the case where the end base is cytosine (c), preferably, the base sequence of the fluorescence quenching probe is designed such that, in the control template or the specimen nucleic acid, a base to be paired with the labeled end cytosine (c) or a base one to three bases away therefrom is guanine (g) when the fluorescence quenching probe is hybridized with the control template or the specimen nucleic acid. A base one base away from the base to be paired with the labeled end cytosine (c) means a base adjacent thereto. Such a probe is generally called a guanine quenching probe and is known as the so-called Qprobe®. When such a guanine quenching probe hybridizes to the specimen nucleic acid, for example, the phenomenon in which emission of the fluorescent substance decreases (fluorescence intensity decreases) as the end cytosine (c) labeled with the fluorescent substance approaches guanine (g) in the control template or the specimen nucleic acid occurs. By using such a probe, hybridization and disassociation can be recognized easily on the basis of the change in fluorescence intensity.

The detection probe may include a phosphate group added to the 3' end thereof. As described later, the control template and the specimen nucleic acid can be prepared by a nucleic acid amplification method such as PCR or the like. At this time, the reaction system for the amplification reaction can also contain the detection probe. In such a case, when the detection probe include a phosphate group added to the 3' end thereof, elongation of the probe itself by the amplification reaction can be sufficiently prevented. Further, similar effects can be obtained when the detection probe include a labeling substance added to the 3' end thereof.

The primer, the detection probe, and the control template may be composed, for example, of nucleotides such as deoxyribonucleotide, ribonucleotide, and the like. Further, they may contain LNA that is an RNA analog, PNA that is a peptide nucleic acid, BNA that is a bridged nucleic acid, and the like.

In the step (a1), the method for amplifying a nucleic acid is not particularly limited and examples thereof include a polymerase chain reaction (PCR) method, a nucleic acid sequence based amplification (NASBA) method, a transcription-mediated amplification (TMA) method, a strand displacement amplification (SDA) method, and the like. Among them, the PCR method is preferred. It is to be noted that conditions for the nucleic acid amplification method are not particularly limited and the nucleic acid amplification can be performed by a conventionally known method.

The step (a2) is an analysis step of performing the melting curve analysis as described above and, for example, performing the so-called Tm analysis is preferred. Hereinafter, the Tm value in the Tm analysis will be described. The absorbance at 260 nm increases as a solution containing a double-stranded DNA is heated, for example. This increase is caused by the fact that the hydrogen bond between both the strands in a double-stranded DNA is unbound by heating, and the double-stranded DNA is dissociated into single-stranded DNAs (i.e., DNA melting). When every double-stranded DNA is dissociated into single-stranded DNAs, the solution exhibits an absorbance about 1.5 times as large as the absorbance at the time when the heating was initiated (the absorbance of the solution containing only the double-stranded DNA), whereby it can be determined that the melting is completed. Based on this phenomenon, a melting temperature Tm is generally defined as a temperature at the time when the amount of increase in absorbance reaches 50% of the total amount of increase in absorbance. The Tm value can be calculated by using, for example, known MELTCALC software (www.meltcalc.com/) or the nearest neighbor method or can be calculated by actual measurement.

In the step (a2), the melting curve analysis can be performed, for example, as follows. That is, the step (a2) is a step of changing the temperature of the reaction system in the step (a1) containing an amplification product of the control template in the presence of the detection probe and measuring a signal value that shows a molten state of a hybridization product between the amplification product and the detection probe.

In the step (a2), the measurement of the signal that shows the molten state of the hybridization product between the amplification product and the detection probe may be the measurement of an absorbance at 260 nm as described above or may be the measurement of the signal from a labeling substance. Specifically, as described above, it is preferable that a labeled probe labeled with the labeling substance is used as the detection probe to perform the measurement of the signal of the labeling substance. The labeled probe can be, for example, a labeled probe that exhibits a signal independently but does not exhibit a signal by hybridization, or a labeled probe that does not exhibit a signal independently but exhibits a signal by hybridization. The former probe does not exhibit a signal when the probe forms a hybridization product (double-stranded DNA) with the amplification product but exhibits a signal when the probe is dissociated from the amplification product by heating. On the other hand, the latter probe exhibits a signal when the probe forms a hybridization product (double-stranded DNA) with the amplification product but the signal is reduced (quenched) when the probe is dissociated from the amplification product by heating. Accordingly, by detecting the signal of the labeling substance, progress of the melting of the hybridization product and the Tm value can be determined as in the case of the measurement of an absorbance at 260 nm. The signal from the labeling substance can be detected under conditions specific to the signal of the labeling substance. The conditions are, for example, an excitation wavelength, a detection wavelength, and the like. It is to be noted that the labeled probe and the labeling substance are the same as those described above.

The timing of addition of the detection probe is not particularly limited as long as the detection probe is added before the step (a2). The detection probe can be added, for example, before, during, or after the amplification step (a1). Above all, for example, it is preferable to add the detection probe to the reaction system before the amplification reaction in the step (a1) because there is no need to expose the reaction system to an external environment for the addition of the detection probe thereto and the amplification reaction in the step (a1) and the melting curve analysis in the step (a2) can be performed continuously. In this case, as described above, the 3' end of the detection probe is preferably modified with a labeling substance or a phosphate group.

In the step (a3), the determination of whether the positive control and the negative control each are positive or negative can be made, for example, as follows. That is, in the melting curve,
when a peak is present at the Tm value (Tm_{C}) of a double-stranded nucleic acid composed of the amplification region of the control template and the detection probe, the positive control is determined as positive (+);
when a peak is not present at the Tm value (Tm_{C}) of a double-stranded nucleic acid composed of the amplification region of the control template and the detection probe, the positive control is determined as negative (-);
when a peak is not present at a temperature other than the Tm value (Tm_{C}) of a double-stranded nucleic acid composed of the amplification region of the control template and the detection probe, the negative control is determined as negative (-); and
when a peak is present at a temperature other than the Tm value (Tm_{C}) of a double-stranded nucleic acid composed of the amplification region of the control template and the detection probe, the negative control is determined as positive (+).

Next, the control detection method of the present invention will be described with reference to an example where the control template and the detection probe shown in FIG. 1 are used and nucleic acid amplification is performed by PCR. The present invention is not limited thereto. Further, conditions for PCR are not particularly limited and PCR can be performed according to a conventionally known method.

First, a reaction solution containing the control template, the primer, and the detection probe is prepared.

The amount of the control template to be added to the reaction solution is not particularly limited. The control template is added to be, for example, preferably 10⁻¹² µmol/L to 10⁻⁷ µmol/L, more preferably 10⁻¹¹ µmol/L to 10⁻⁸ µmol/L, and particularly preferably 10⁻¹⁰ µmol/L to 10⁻⁹ µmol/L.

The amount of the primer to be added to the reaction solution is not particularly limited. The primer is added to be, for example, preferably 0.01 µmol/L to 10 µmol/L, more preferably 0.05 µmol/L to 5 µmol/L, and particularly preferably 0.1 µmol/L to 1 µmol/L. The type of the primer is not particularly limited. It is preferable to use two or more primers as described above, and to add the respective primers in the aforementioned range.

The amount of the detection probe to be added to the reaction system is not particularly limited. The detection probe is added to be, for example, preferably 10 nmol/L to 400 nmol/L and more preferably 20 nmol/L to 200 nmol/L.

Other composition components in the reaction solution are not particularly limited. Conventionally known components can be used as the other composition components and the proportions thereof also are not particularly limited. Examples of the components include polymerase such as DNA polymerase and the like; nucleotide (nucleoside triphosphate); solvents; and the like.

The DNA polymerase is not particularly limited and, for example, a conventionally known thermoduric bacteria-derived polymerase can be used. Specifically, for example, *Thermus aquaticus*-derived DNA polymerase (USP 4,889,818 and USP 5,079,352) (product name: Taq polymerase), *Thermus thermophilus*-derived DNA polymerase (WO 91/09950) (rTth DNA polymerase), *Pyrococcus furiosus*-derived DNA polymerase (WO 92/9689) (Pfu DNA polymerase; produced by Stratagene), and *Thermococcus litoralis*-derived DNA polymerase (EP 0455430) ("Vent™"; produced by New England Biolabs) are commercially available. Particularly, *Thermus aquaticus*-derived thermostable DNA polymerase is preferred.

The proportion of DNA polymerase to be added to the reaction solution is not particularly limited however is, for example, 1 U/mL to 100 U/mL, preferably 5 U/mL to 50 U/mL, and more preferably 20 U/mL to 30 U/mL. With respect to the unit of activity (U) of DNA polymerase, generally, 1 U denotes the activity that allows all 10 nmol of nucleotide to be taken into an acid-insoluble sediment in 30 minutes at 74°C in a reaction solution for activity measurement, with an activated salmon sperm DNA being used as a template primer. The composition of the reaction solution for activity measurement is, for example, 25 mmol/L TAPS buffer (pH 9.3, 25°C), 50 mmol/L KCl, 2 mmol/L MgCl₂, 1 mmol/L mercaptoethanol, 200 µmol/L dATP, 200 µmol/L dGTP, 200 µmol/L dTTP, 100 µmol/L [α⁻³²P] dCTP, and 0.25 mg/mL activated salmon sperm DNA.

Normally, examples of the nucleoside triphosphate include dNTP (dATP, dCTP, dGTP, and dTTP or dUTP). The proportion of dNTP to be added to the reaction solution is not particularly limited however is, for example, 0.01 mmol/L to 1 mmol/L, preferably 0.05 mmol/L to 0.5 mmol/L, and more preferably 0.1 mmol/L to 0.3 mmol/L.

Examples of the solvents include Tris-HCl, Tricine, MES, MOPS, HEPES, CAPS, and the like. Commercially available PCR buffer solutions or buffer solutions of commercially available PCR kits can be used.

Furthermore, the reaction solution further may contain, heparin, betaine, KCl, MgCl₂, MgSO₄, glycerol, and the like. The proportions thereof to be added can be set in ranges in which the PCR reaction is not impaired.

The total volume of the reaction solution is not particularly limited and can be determined suitably according to, for example, a device to be used (thermal cycler). It is normally 1 µL to 500 µL and preferably 10 µL to 100 µL.

Subsequently, amplification of the control template is performed using the reaction solution by PCR. The PCR includes, for example, three steps: (1) dissociation of a double-stranded nucleic acid into single-stranded nucleic acids, (2) annealing of primers, and (3) elongation of the primers (polymerase reaction). Conditions for the respective steps are not particularly limited. The step (1) is performed, for example, preferably at 90°C to 99°C for 1 second to 120 seconds, and more preferably at 92°C to 95°C for 1 second to 60 seconds. The step (2) is performed, for example, preferably at 40°C to 70°C for 1 second to 300 seconds, and more preferably at 50°C to 70°C for 5 seconds to 60 seconds. The step (3) is performed, for example, preferably at 50°C to 80°C for 1 second to 300 seconds, and more preferably at 50°C to 75°C for 5 seconds to 60 seconds. Furthermore, the number of cycles also is not particularly limited but preferably is at least 30, with the aforementioned three steps (1) to (3) being considered as one cycle. The upper limit thereof, in total, is not particularly limited however is, for example, 100 cycles or less, preferably 70 cycles or less, and further preferably 50 cycles or less. The change in temperature in each step can be controlled automatically using, for example, a thermal cycler.

Then, using the reaction system after the amplification reaction, a melting curve analysis is performed.

In the melting curve analysis, for example, the temperature of the reaction system after the amplification step is changed, the signal value that shows the molten state of the hybridization product between the amplification product and the detection probe is measured, and the variation in the signal value accompanying the change in the temperature is analyzed. The temperature showing the great variation in the signal value is the Tm value of the hybridization product between the amplification product and the detection probe, and it can be determined that the peak of the variation in the signal value is present at that temperature. Further, in the case where the temperature showing the great variation in the signal value is not present, it can be determined that the peak of the variation in the signal value is not present. The determination of the control is made in the determination step (a3) that will be described later on the basis of the presence or absence of the peak and the temperature (Tm value) showing the peak.

The hybridization product between the amplification product and the detection probe can be formed by changing the temperature of the reaction solution in the presence of the detection probe. In this case, as described above, it is preferable that the amplification reaction is carried out with respect to the reaction solution to which the detection probe is added beforehand, and then the temperature of the reaction solution is changed.

The hybridization product between the amplification product and the detection probe is formed, for example, as follows. First, the amplification product that is a double-stranded DNA is dissociated into single-stranded DNAs, and then the single-stranded DNA is hybridized with the detection probe.

The dissociation of the amplification product can be carried out, for example, by heating the reaction solution. The heating temperature is not particularly limited as long as it allows the amplification product to be dissociated. It is, for example, 85°C to 95°C. The heating time also is not particularly limited and normally is 1 second to 10 minutes and preferably 1 second to 5 minutes.

The dissociated single-stranded DNA can be hybridized with the detection probe, for example, by decreasing the heating temperature employed for dissociation after the heating treatment for dissociation. In other words, it can be performed, for example, by decreasing the temperature of the reaction solution. The temperature condition is, for example, 40°C to 50°C. Further, the treatment time at the aforementioned temperature condition is, for example, 1 second to 600 seconds although it is not particularly limited.

Then, as described above, the temperature of the reaction solution is changed and thereby the signal value that shows the molten state of the hybridization product between the amplification product and the detection probe is measured. Specifically, for example, the reaction solution (the hybridization product) is heated, and the variation in the signal value accompanying the temperature rise is measured.

The signal value may be measured by measuring an absorbance at 260 nm using an unlabeled probe. As described above, it is preferable to measure the signal value using the aforementioned labeled probe by measuring the signals of the respective labeling substances. As described above, the labeled probe can be, for example, a labeled probe that exhibits a signal independently but does not exhibit a signal by hybridization, or a labeled probe that does not exhibit a signal independently but exhibits a signal by hybridization. The former probe does not exhibit a signal when the probe forms a hybridization product (double-stranded nucleic acid) but exhibits a signal when the probe is dissociated by heating. Accordingly, when such a probe is used, for example, the hybridization product in which the fluorescence has decreased (or been quenched) is heated gradually and the increase in fluorescence intensity accompanying the temperature rise is measured. As for the former probe, for example, the aforementioned guanine quenching probe can be employed. The latter probe exhibits a signal when the probe forms a hybridization product (double-stranded nucleic acid) but the signal is reduced (quenched) when the probe is dissociated by heating. Accordingly, when such a probe is used, contrary to the case where the former probe is used, the hybridization product is heated gradually and the amount of the decrease in fluorescence intensity accompanying the temperature rise is measured.

The temperature range in which the variation in the signal value is to be measured is not particularly limited. The start temperature is, for example, room temperature to 85°C and preferably 25°C to 70°C, while the end temperature is, for example, 40°C to 105°C. Furthermore, the rate of temperature rise is not particularly limited however is, for example, 0.1 °C/sec to 20 °C/sec and preferably 0.3 °C/sec to 5 °C/sec.

Next, the Tm value showing the great variation (peak) in the signal value is determined by analyzing the variation in the signal value accompanying the change in temperature based on the signal value measured. The variation in the signal value can be analyzed, for example, by calculating the amount of change in the signal value (F) per unit time (t). When the signal value is increased due the melting of the hybridization product (i.e., dissociation of the hybridization product into single-stranded nucleic acids), for example, the amount of increase per unit time (t) or its negative differential value (-dF increase amount / dt) of the signal value (F) is calculated, and the temperature at which the lowest value is obtained can be determined as the Tm value. Alternatively, for example, the amount of increase per unit time (t) or its differential value (dF increase amount / dt) of the signal value (F) is calculated, and the temperature at which the highest value is obtained can be determined as the Tm value. In contrast, when the signal value is decreased due the melting of the hybridization product (i.e., dissociation of the hybridization product into single-stranded nucleic acids), for example, the Tm value can be determined by calculating the amount of decrease in the signal value (F) per unit time (t).

The method, in which the signal value during hybridization is measured and the variation in the signal value is analyzed, may be employed instead of the method, in which the temperature of the reaction solution is raised and the variation in the signal value accompanying the temperature rise is measured as described above. In other words, when the temperature of the reaction system is decreased to form the hybridization product, the variation in the signal accompanying the temperature decrease may be measured.

The variation in the signal value can be analyzed by making a graph plotting the relationship between the temperature and the variation in the signal value. However, in the melting curve analysis, it is not essential to prepare such a graph.

Subsequently, the determination of the positive control and the negative control is made on the basis of the results of the melting curve analysis.

When the temperature of the peak obtained in the melting curve analysis is the Tm value (Tm_{C}) of a double-stranded nucleic acid composed of the amplification region of the control template nucleic acid and the detection probe, it tells that nucleic acid amplification occurs in the reaction solution. In other words, it can be determined that the positive control is positive (+). In contrast, when the peak is not detected in the melting curve analysis, it tells that nucleic acid amplification does not occur in the reaction system. In other words, it can be determined that the positive control is negative (-). Further, in the melting curve analysis, when the peak is also detected at a temperature other than the Tm value (Tm_{C}) of a double-stranded nucleic acid composed of the amplification region of the control template and the detection probe, it tells that the reaction system is contaminated with undesired nucleic acid and that nucleic acid amplification occurs with the undesired nucleic acid being used as a template. In other words, it can be determined that the negative control is positive (+). On the other hand, in the melting curve analysis, when the peak is not detected at a temperature other than the Tm value (Tm_{C}) of a double-stranded nucleic acid composed of the amplification region of the control template and the detection probe, it tells that the reaction system is not contaminated with undesired nucleic acid. In other words, it can be determined that the negative control is negative (-). On the basis of these results, for example, in the case where the positive control is determined as positive (+) and the negative control is determined as negative (-), an objective analysis such as the detection of whether or not amplification occurs may be performed separately with respect to a reaction solution in which an amplification reaction is performed with a specimen nucleic acid. On the other hand, in the case where the positive control is determined as negative (-) or the negative control is determined as positive (+), it can be determined that the analysis of the reaction system of the specimen nucleic acid is unavailable, and the measurement may be carried out again.

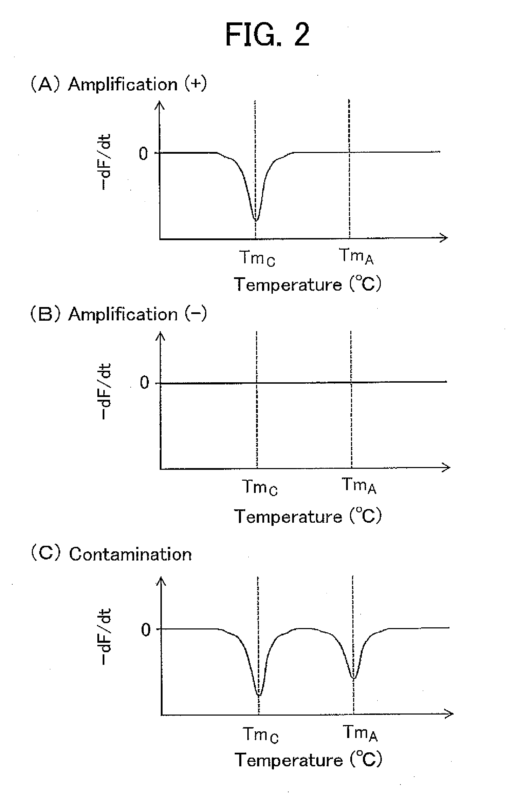

FIG. 2 shows examples of the graphs plotting the relationship between the variation in the signal value (-dF/dt) and the temperature in the melting curve analysis using the control template and the detection probe shown in FIG. 1. It is to be noted that these are merely illustrative and do not limit the present invention. As the detection probe, a probe that exhibits a signal when it is dissociated but the signal thereof is suppressed when it forms a double-stranded nucleic acid is used. In FIG. 2, the horizontal axis indicates temperature (°C). The vertical axis indicates the variation in the signal value, and "-d signal change amount / dt" (-dF/dt), which is the differential value of the signal change amount is used as the unit. In FIG. 2A, since the peak is detected only at the Tm value (Tm_{C}) of a double-stranded nucleic acid composed of the amplification region of the control template and the detection probe, it can be determined that amplification occurs, i.e., it can be determined that the positive control is positive (+). Further, since the peak is not detected besides the aforementioned peak, it can be determined that the reaction system is not contaminated with undesired nucleic acid, i.e., it can be determined that the negative control is negative (-). In FIG. 2B, since the peak is not detected at any temperature, it can be determined that amplification does not occur, i.e., it can be determined that the positive control is negative (-). In FIG. 2C, since the peak is detected not only at the Tm value (Tm_{C}) of a double-stranded nucleic acid composed of the amplification region of the control template and the detection probe but also at the Tm value (Tm_{A}) of a double-stranded nucleic acid composed of the target sequence and the detection probe, it can be determined that the reaction system is contaminated with undesired nucleic acid, i.e., it can be determined that the negative control is positive (+).

As shown in FIG. 2C, in the case of the negative control (+), the peak is present at the temperature that is different from the temperature at which the peak of the positive control (+) is detected. Therefore, from the following reasons, it is preferable to minimize the amount of the control template to be added to the reaction system. When the analysis is performed under this condition, for example, when the peak at the Tm_{A} that indicates the negative control (+) is lower than the peak at the Tm_{C} that indicates the positive control (+), remeasurement caused by negative control (+) can be omitted as an undesired nucleic acid does not affect the analysis. In this case, the amount of the control template to be added to the reaction system is, for example, preferably 10⁻¹² µmol/L to 10⁻⁷ µmol/L, more preferably 10⁻¹¹ µmol/L to 10⁻⁸ µmol/L, and particularly preferably 10⁻¹⁰ µmol/L to 10⁻⁹ µmol/L.

Further, in the present invention, for example, the melting curve analysis may be performed in the presence of the detection probe, control oligonucleotide, and the control probe consisting of a base sequence complementary to the control oligonucleotide in the step (a2). For example, with respect to the control oligonucleotide and the control probe, the Tm value of a double-stranded nucleic acid composed of the control oligonucleotide and the control probe is known and the signal intensity in the reaction system also is known.

Generally, the melting curve analysis is performed using a device provided with a temperature control unit and a signal detection unit. However, with respect to such a device, there is a case in which the temperature control unit cannot control the temperature correctly and the signal detection unit cannot detect the signal correctly. Therefore, in the former case, there is a possibility that the peak of the signal is detected at the temperature that is different from the actual temperature. In the latter case, there is a possibility that the peak that is different from the actual value is detected. From these reasons, it is important to check whether or not the temperature control unit and the signal detection unit function correctly in the device. Hence, in the step (a2), when both the control oligonucleotide and the control probe are present, since the control probe forms a hybridization product with the control oligonucleotide, the melting curve analysis thereof also can be performed using the same reaction system. Further, since the Tm value of a double-stranded nucleic acid composed of the control oligonucleotide and the control probe is known as described above, the determination of whether or not the temperature control unit functions normally can be made on the basis of whether or not a peak of the signal is present at the known Tm value with reference to the reaction system. That is, for example, in the case where the known Tm value is 60°C, it can be determined that the temperature control unit functions normally when the peak is detected at around 60°C indicated by the device, and it can be determined that the temperature control unit does not function normally when the peak is not detected at around 60°C but detected at other temperature. Furthermore, by performing the melting curve analysis of the control oligonucleotide and the control probe, it can be determined, for example, whether or not the signal detection unit functions normally. That is, for example, by detecting the noise in the melting curve analysis of the control oligonucleotide and the control probe, the determination of whether or not an objective peak is detected due to the defective detection of the signal can be made. Accordingly, the erroneous determination can be prevented. In the present invention, hereinafter, the control oligonucleotide may also be referred to as "Tm control oligonucleotide" and the control probe may also be referred to as "Tm control probe".

Preferably, the Tm control probe is a labeled probe having a labeling substance. Since the reaction system contains both the detection probe and the Tm control probe, the detection probe and the Tm control probe are preferably labeled with different labeling substances, respectively. Examples of the labeling substances include those described above.

The timing of the addition of the Tm control oligonucleotide and the Tm control probe is not particularly limited, and is, for example, same as that described for the detection probe. For example, the Tm control oligonucleotide and the Tm control probe are preferably added to the reaction system in advance of the step (a1).

When the amplification reaction of the step (a1) is carried out in the presence of the Tm control oligonucleotide and the Tm control probe, in order to prevent the elongation of the Tm control oligonucleotide and the Tm control probe from themselves, the Tm control oligonucleotide and the Tm control probe each further may include a phosphate group added to the 3' end thereof. Further, the Tm control probe may include a labeling substance added to the 3' end thereof. This also prevents the elongation of the Tm control probe itself.

The complementarity between the Tm control oligonucleotide and the Tm control probe is preferably 90% or more and more preferably 100%. Preferably, the Tm control oligonucleotide and the Tm control probe have the same length. The length is, for example, preferably 5 to 40 bases, more preferably 10 to 30 bases, and yet more preferably 15 to 25 bases.

In order to allow the Tm control probe to be hybridized with the Tm control oligonucleotide, for example, it is preferable that the complementarity of the Tm control probe to the Tm control oligonucleotide is higher than that of the Tm control probe to other nucleic acid sequences present in the reaction system. Further, in order to allow the Tm control oligonucleotide to be hybridized with the Tm control probe, it is preferable that the complementarity of the Tm control oligonucleotide to the Tm control probe is higher than that of the Tm control oligonucleotide to other nucleic acid sequences present in the reaction system. For example, it is preferable that the Tm control probe and the Tm control oligonucleotide each are hard to hybridize with the aforementioned other nucleic acid sequences, and it is more preferable that the Tm control probe and the Tm control oligonucleotide each do not hybridize with the other nucleic acid sequences. Examples of the other nucleic acid sequences include the detection probe, the specimen nucleic acid and its target sequence, the control template nucleic acid and its amplification region, the primer, and the like. The complementarity of the foregoing Tm control probe to the other nucleic acid sequences and the complementarity of the Tm control oligonucleotide to the other nucleic acid sequences each are, for example, preferably 30% or less and more preferably 20% or less.

For example, it is preferable that the Tm value (Tmₜ) of a double-stranded nucleic acid composed of the Tm control oligonucleotide and the Tm control probe is different from the Tm value (Tm_{C}) of a double-stranded nucleic acid composed of the amplification region of the control template nucleic acid and the detection probe. Further, it is preferable that the Tm value (Tmₜ) of a double-stranded nucleic acid composed of the Tm control oligonucleotide and the Tm control probe is different from the Tm value (Tm_{A}) of a double-stranded nucleic acid composed of the target sequence and the detection probe.

When the Tm control oligonucleotide and the Tm control probe are used together, it is preferable that the step (a3) further judges whether or not the temperature control in the step (a2) is carried out correctly on the basis of a peak in the melting curve.

### < Amplification Method >

As described above, the amplification method of the present invention is a method for amplifying a target sequence of a specimen nucleic acid in a reaction system. The amplification method includes the following steps (A) and (B):
(A) detecting controls showing amplification efficiency with respect to a reaction system containing the control template nucleic acid, the primer, and the detection probe by the control detection method of the present invention; and
(B) amplifying the target sequence of the specimen nucleic acid, including the following step (b1):
   (b1) amplifying the target sequence of the specimen nucleic acid in a reaction system containing the specimen nucleic acid, the primer, and the detection probe using the primer.

The amplification method of the present invention is a method in which controls are examined by the control detection method of the present invention. Except for this, steps, conditions, and the like of the amplification method are not particularly limited. Further, the control detection method of the present invention is as described above unless otherwise stated, and can be applied to the amplification method of the present invention. The step (A) includes the same steps (a1) to (a3) as those used in the control detection method of the present invention.

The specimen reaction system to be used in the step (b1) is not particularly limited as long as it contains an objective specimen nucleic acid. Conditions of the specimen reaction system are preferably the same as those of the control reaction system. The specimen reaction system may or may not contain the control template, for example. As described above, it is preferable that the step (b1) is performed in the same manner as the step (a1).

Preferably, the step (B) further includes the following steps (b2) and (b3). The step (b2) can be performed in the same manner as the step (a2).
(b2) performing a melting curve analysis in the presence of the detection probe using the reaction system obtained in the step (b1); and
(b3) determining whether or not the target sequence of the specimen nucleic acid is amplified on the basis of a peak in the melting curve.

Preferably, the step (b3) is performed in the case where the positive control is determined as positive (+) and the negative control is determined as negative (-) in the step (a3) in the step (A). In the case where the positive control is determined as negative (-) or the negative control is determined as positive (+) in the step (a3), it is preferable to omit the step (b3) and to carry out measurement again.

In the step (b3), when a peak is present at the Tm value (Tm_{A}) of a double-stranded nucleic acid composed of the target sequence and the detection probe, it is determined that the target sequence of the specimen nucleic acid is amplified, and when a peak is not present at the Tm value (Tm_{A}) of a double-stranded nucleic acid composed of the target sequence and the detection probe, it is determined that the target sequence of the specimen nucleic acid is not amplified.

Preferably, the step (A) for the control reaction system and the step (B) for the specimen reaction system are performed simultaneously under the same conditions. Specifically, it is preferable to perform the steps (a1) and (b1) simultaneously under the same conditions and to perform the steps (a2) and (b2) simultaneously under the same conditions.

The specimen nucleic acid is not particularly limited and may be a single-stranded nucleic acid or a double-stranded nucleic acid. Examples of the specimen nucleic acids include DNAs; RNAs such as total RNA, mRNA, and the like; and the like. Further, examples of the specimen nucleic acids include nucleic acids contained in samples such as a biological sample and the like. The specimen nucleic acid in the sample may be a nucleic acid inherently contained in a biological sample. Further, the specimen nucleic acid may be an amplification product amplified by the nucleic acid amplification method with the nucleic acid in the biological sample being used as a template, for example. Specific examples thereof include an amplification product obtained through amplification by the nucleic acid amplification method with DNA inherently contained in the biological sample being used as a template; cDNA produced from RNA inherently contained in the biological sample by reverse transcription PCR (RT-PCR); an amplification product obtained through amplification by the nucleic acid amplification method with the cDNA being used as a template; and the like. The length of the amplification product is not particularly limited, however is, for example, 50 to 1000 bases and preferably 80 to 200 bases.

The sample containing the specimen nucleic acid is not particularly limited, and examples thereof include whole blood, blood cell, blood plasma, blood serum, cells in the mouth such as oral mucosa, somatic cells such as nails and hairs, germ cells, expectoration, amniotic fluid, paraffin-embedded tissue, urine, gastric juice, gastric lavage fluid, and suspensions thereof.

The amount of the specimen nucleic acid to be added to the reaction system is not particularly limited. The specimen nucleic acid is added to be, for example, preferably 1 µg/L to 1,000,000 µg/L, more preferably 10 µg/L to 100,000 µg/L, and particularly preferably 100 µg/L to 10,000 µg/L.

Further, in the present invention, for example, the melting curve analysis may be performed in the step (b2) using the reaction system for amplifying the specimen nucleic acid in the presence of the detection probe, the Tm control oligonucleotide, and the Tm control probe consisting of a base sequence complementary to the Tm control oligonucleotide. The Tm control oligonucleotide and the Tm control probe are the same as those described above. Also with respect to these, for example, as described above, the determination of whether or not the temperature control is carried out normally can be made by performing the melting curve analysis.

### <Melting Curve Analysis Method>

As described above, the melting curve analysis method of the present invention is a melting curve analysis method of a reaction system containing a specimen nucleic acid. The melting curve analysis method includes the following steps (A) and (C):
(A) detecting controls showing amplification efficiency with respect to a reaction system containing the control template nucleic acid, the primer, and the detection probe by the control detection method of the present invention; and
(C) performing a melting curve analysis, including the following steps (c1) and (c2):
   (c1) amplifying a target sequence of the specimen nucleic acid in a reaction system containing the specimen nucleic acid, the primer, and the detection probe using the primer; and
   (c2) performing the melting curve analysis in the presence of the detection probe using the reaction system obtained in the step (c1).

The melting curve analysis method of the present invention is a method in which controls are examined by the control detection method of the present invention. Except for this, steps, conditions, and the like of the melting curve analysis method are not particularly limited. The step (A) includes, for example, the same steps (a1) to (a3) as those used in the control detection method of the present invention.

The melting curve analysis method is preferably used for a polymorphism detection method, for example. Therefore, the melting curve analysis method of the present invention also can be called as a polymorphism detection method. When the melting curve analysis method of the present invention is the polymorphism detection method, for example, it is preferable that a target sequence of the specimen nucleic acid contains an objective target site that shows polymorphism, the detection probe is a probe capable of hybridizing to a region containing the target site in the target sequence, and the step (C) further includes the following step (c3):
(c3) detecting the polymorphism on the basis of a peak in the melting curve.

Preferably, the step (A) for the control reaction system and the step (C) for the specimen reaction system are performed simultaneously. Specifically, it is preferable to perform the steps (a1) and (c1) simultaneously and to perform the steps (a2) and (c2) simultaneously. In the step (C), the step (c1) is the same as the step (b1) in the amplification method of the present invention and the step (c2) is the same as the step (b2) in the amplification method of the present invention.

In the present invention, for example, the detection probe may be designed on the assumption that a polymorphism of the target site is a wild-type (normal-type) or that a polymorphism of the target site is a mutant-type. That is, the detection probe may be designed such that a base corresponding to the target site is a base complementary to a wild-type base or a base complementary to a mutant-type base. In the former case, the detection probe matches the wild-type target site and the detection probe mismatches the mutant-type target site. In the latter case, the detection probe mismatches the wild-type target site and the detection probe matches the mutant-type target site. In this manner, in the target site, the difference between the base of the target sequence and the base of the detection probe occurs depending on whether the target site is a wild-type or a mutant-type. Therefore, it is possible to detect whether the polymorphism of the target site is a wild-type or a mutant-type. The complementarity between a region of the detection probe to be hybridized with the target sequence and a region of the target sequence to be hybridized with the detection probe, excluding the target sequence, is preferably 90% to 100% and more preferably 100%.

In the melting curve analysis, the case of a perfectly complementary hybrid (perfect match) results in a higher Tm value indicating dissociation than that obtained in the case of a hybrid in which a single base or more than a single base is different (mismatch). Accordingly, when the Tm value obtained in the case of a perfectly complementary hybrid and the Tm value obtained in the case of a hybrid in which a single base or more than a single base is different are determined beforehand with respect to the detection probe, the polymorphism at the target site can be determined.

For example, when the detection probe is a probe complementary to a region containing a mutant-type base site in the target sequence, for example, the polymorphism can be determined as follows in the step (c3). That is, for example, when a peak is present at the Tm value (Tm_{MT}) of a double-stranded nucleic acid composed of the target sequence containing the mutant-type base site and the detection probe, it is determined that the base site of the specimen nucleic acid is a mutant-type; and when a peak is present at the Tm value (Tm_{WT}) of a double-stranded nucleic acid composed of the target sequence containing the wild-type base site and the detection probe, it is determined that the base site of the specimen nucleic acid is a wild-type. In this case, the temperature of the Tm_{MT} is higher than that of the Tm_{WT}.

Further, when the detection probe is a probe complementary to a region containing a wild-type base site in the target sequence, the polymorphism can be determined as follows in the step (c3). That is, for example, when a peak is present at the Tm value (Tm_{WT}) of a double-stranded nucleic acid composed of the target sequence containing the wild-type base site and the detection probe, it is determined that the base site of the specimen nucleic acid is a wild-type; and when a peak is present at the Tm value (Tm_{MT}) of a double-stranded nucleic acid composed of the target sequence containing the mutant-type base site and the detection probe, it is determined that the base site of the specimen nucleic acid is a mutant-type. In this case, the temperature of the Tm_{WT} is higher than that of the Tm_{MT}.

Further, in the present invention, for example, the melting curve analysis may be performed in the step (c2) using the reaction system for amplifying the specimen nucleic acid in the presence of the detection probe, the control oligonucleotide, and the control probe consisting of a base sequence complementary to the control oligonucleotide. The control oligonucleotide and the control probe are the same as those described above. Also with respect to these, for example, as described above, the determination of whether or not the temperature control is carried out normally can be made by performing the melting curve analysis.

### <Control Detection Reagent>

The control detection reagent of the present invention is a control detection reagent used for the control detection method of the present invention. The control detection reagent contains:
a control template nucleic acid, wherein
the control template nucleic acid can be amplified by a primer capable of amplifying an objective target sequence,
an amplification region of the control template nucleic acid amplified by the primer can be hybridized with a detection probe capable of hybridizing to the target sequence, and
a Tm value (Tm_{C}) of a double-stranded nucleic acid composed of the amplification region of the control template nucleic acid and the detection probe is different from a Tm value (Tm_{A}) of a double-stranded nucleic acid composed of the target sequence and the detection probe.

The control detection reagent of the present invention contains the aforementioned control template. Except for this, the configuration thereof is not limited at all.

The control detection reagent of the present invention may contain, for example, a primer and a detection probe besides the control template. When the control detection reagent of the present invention contains the primer and the detection probe, the control detection reagent can be used, for example, for amplification and a melting curve analysis of the specimen nucleic acid. In this case, a specimen reaction system containing the specimen nucleic acid may contain, for example, the control template eventually. Although the specimen reaction system contains the control template, as described above, the Tm value (Tm_{A}) of a double-stranded nucleic acid composed of the target sequence of the specimen nucleic acid and the detection probe is different from the Tm value (Tm_{C}) of a double-stranded nucleic acid composed of the amplification region of the control template and the detection probe. Therefore, amplification and the melting curve analysis of the specimen reaction system can be performed in the same manner as in the case where the specimen reaction system does not contain the control template.

The control detection reagent of the present invention further may contain various components such as polymerase, dNTP, and the like. Further, the control detection reagent of the present invention may be a kit used for the control detection method of the present invention in which each of the components is contained in a separate container. Preferably, the kit has, for example, an instruction manual.

### <Analysis Reagent>

The analysis reagent of the present invention is an analysis reagent used for the melting curve analysis method of the present invention. The analysis reagent contains:
the control detection reagent of the present invention; and
an amplification reaction reagent for a specimen nucleic acid containing the primer and the detection probe.
The analysis reagent of the present invention may be a kit used for the melting curve analysis method of the present invention in which each of the components is contained in a separate container. Further, the analysis reagent of the present invention may be a kit in which the control detection reagent and the amplification reaction reagent each are contained in a separate container. Preferably, these kits have, for example, instruction manuals.

### <Design Method>

The design method of the present invention is a method for designing a control template nucleic acid for detecting controls showing amplification efficiency of a reaction system when a target sequence of a specimen nucleic acid is amplified,
the control template nucleic acid being a template nucleic acid capable of detecting both a positive control showing an occurrence of an amplification reaction in the reaction system and a negative control showing an occurrence of contamination of the reaction system with undesired nucleic acid, the method including a step of:
designing a base sequence of the control template nucleic acid such that the control template nucleic acid can be amplified by a primer capable of amplifying the target sequence,
an amplification region of the control template nucleic acid amplified by the primer can be hybridized with a detection probe capable of hybridizing to the target sequence, and
a Tm value (Tm_{C}) of a double-stranded nucleic acid composed of the amplification region and the detection probe is different from a Tm value (Tm_{A}) of a double-stranded nucleic acid composed of the target sequence and the detection probe.

### <Use>

The use of the present invention is the use of a control template nucleic acid for detecting controls showing amplification efficiency of a reaction system when a target sequence of a specimen nucleic acid is amplified, the control template nucleic acid consisting of a base sequence designed such that
the control template nucleic acid can be amplified by a primer capable of amplifying the target sequence,
an amplification region of the control template nucleic acid amplified by the primer can be hybridized with a detection probe capable of hybridizing to the target sequence, and
a Tm value (Tm_{C}) of a double-stranded nucleic acid composed of the amplification region and the detection probe is different from a Tm value (Tm_{A}) of a double-stranded nucleic acid composed of the target sequence and the detection probe, wherein
the control template nucleic acid is used as a control template nucleic acid for detecting, in one reaction system, a positive control showing an occurrence of an amplification reaction in the reaction system and a negative control showing an occurrence of contamination of the reaction system with undesired nucleic acid together.

### <Melting Curve Analysis Method>

The melting curve analysis method of the present invention is a melting curve analysis method for a reaction system containing a specimen nucleic acid. The melting curve analysis method includes a step of: performing the melting curve analysis with reference to the reaction system containing the specimen nucleic acid in the presence of a detection probe, a control oligonucleotide, and a control probe, wherein
the detection probe is a probe capable of hybridizing to a target sequence of the specimen nucleic acid, and
the control probe has a sequence complementary to the control oligonucleotide.

As described above, the present invention achieves, in the same reaction system, not only the analysis of the specimen nucleic acid and the detection probe but also determination of whether or not the temperature control and the signal detection are carried out normally. It is to be noted that the detection probe, the control oligonucleotide, and the control probe are, for example, the same as those described above and the melting curve analysis and the like also can be performed in the same manner as described above unless otherwise stated.

The present invention may include, for example, an amplification step of amplifying a target sequence of the specimen nucleic acid in advance of the analysis step. The amplification step of the specimen nucleic acid can be performed in the same manner as described above, for example.

Next, Examples of the present invention are described. However, the present invention is not limited by the Examples.

### Examples

### Example A1

HPV-positive panel blood plasma was purified by eluting with a TE solution using a nucleic acid purification kit (product name: QIAamp). The purified solution was further diluted 2000-fold to prepare a blood plasma-derived nucleic acid sample. On the other hand, the polynucleotide represented by SEQ ID NO: 1 was inserted into a pT7 plasmid vector as an insertion sequence to prepare a recombinant plasmid. The recombinant plasmid was diluted with a TE solution to prepare a plasmid solution.

Insertion sequence (SEQ ID NO: 1)

5'-tcacctctgcctaatcatctcattttcatgtcctatgttcaatcttccaagctgtgccttgggtggctttgTTgc
atTgacattgacccgtataaagaatttggagcttctgtggagttactctcttttttgccttctgacttctttcc-3'

Then, 1 µL of the blood plasma-derived nucleic acid sample was added to 49 µL of the following reaction solution and PCR was carried out using a thermal cycler (product name: Mastercycler ep gradient S, produced by Eppendorf). Conditions for PCR were as follows. That is, after treating at 50°C for 2 minutes and 95°C for 2 minutes, one cycle of treatment at 95°C for 10 seconds, at 54°C for 10 seconds, and 72°C for 30 seconds was repeated for 50 cycles. Further, the tube containing the PCR reaction solution was transferred to iCycler (product name, produced by Bio-Rad Laboratories, Inc.), and treated at 95°C for 1 second and at 40°C for 60 seconds. Thereafter, the temperature was raised from 40°C to 80°C at a temperature rise rate of 1°C/sec. During the temperature rise, the change in fluorescence intensity at each temperature was measured and the Tm analysis was performed. It is to be noted that, the detection target was the following probe 1, the excitation light wavelength was at 370 nm to 415 nm and the detection wavelength was at 445 nm to 480 nm. Further, PCR and Tm analysis were performed in the same manner as described above except that 1 µL of distilled water instead of the nucleic acid sample was added to 49 µL of the following reaction solution. In the following reaction solution, F primer represents a forward primer and R primer represents a reverse primer (hereinafter, the same applies).

**[Table 1]**

| (PCR reaction solution: µL) | |
|---|---|
| Distilled water | 34.025 |
| 40w/v% Glycerol | 3.125 |
| 10 × Gene Taq Universal Buffer ^{*1} | 5 |
| 100 mmol/L MgCl₂ | 0.75 |
| 10 mmol/L dNTP | 1 |
| 2 U/µL uracil-N-glycosylase | 0.1 |
| 100 µmol/L F primer 1 | 0.5 |
| 100 µmol/L R primer 3 | 0.25 |
| 100 µmol/L F primer 3 | 0.5 |
| 100 µmol/L R primer 2 | 0.25 |
| 5 µmol/L probe 1 | 0.5 |
| 5 µmol/L probe 2 | 0.5 |
| 5 µmol/L probe 3 | 0.25 |
| 5 U/µL GeneTaq FP ^{*1} | 0.25 |
| Plasmid solution | 1 |
| 5 µmol/L Higher order structure inhibitory oligonucleotide | 1 |
| Total | 49 µL |

| | |
|---|---|
| *1 produced by Nippon Gene Co., Ltd. | |

F primer 1 (SEQ ID NO: 2)
   5'-cataagaggactcttggact-3'
R primer 3 (SEQ ID NO: 3)
   5'-atttatgcctacagcctcc-3'
F primer 3 (SEQ ID NO: 4)
   5'-tcacctctgcctaatcatctc-3'
R primer 2 (SEQ ID NO: 5)
   5'-ggaaagaagtcagaaggcaa-3'
Probe 1 (SEQ ID NO: 6)
   5'-atgtccatgccccaaagccacc-(Pacific Blue)-3'
Probe 2 (SEQ ID NO: 7)
   5'-atgtccatgTccTaaagccacc-(BODIPYFL)-3'
Probe 3 (SEQ ID NO: 8)
   5'-aTcAttaacctaatctcctccc-(TAMRA)-3'
Inhibitory oligonucleotide (SEQ ID NO: 9)
   5'-aggcttgaacagtaggacatgaacaAgagatga-P-3'

The blood plasma-derived nucleic acid sample has the sequence represented by SEQ ID NO: 10 (ggtggctttgGGgcatGgacat). In the PCR, the region containing this sequence was amplified. The aforementioned sequence is perfectly complementary (perfect match) to the probe 1. On the other hand, the recombinant plasmid has the sequence represented by the underlined part of SEQ ID NO: 1, i.e., the sequence represented by SEQ ID NO: 45 (ggtggctttgTTgcatTgacat). In the PCR, the region containing this sequence was amplified. In the sequence, three bases (T) written in capital letters are bases different from the base sequence of SEQ ID NO: 10, and the sequence is partially non-complementary (mismatch) to the probe 1.

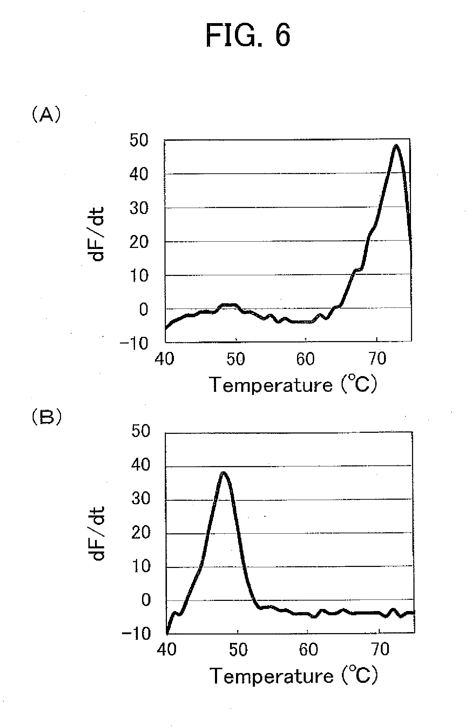

The results thereof are shown in FIGs. 6A and 6B. FIGs. 6A and 6B show graphs of the Tm analysis indicating the change in fluorescence intensity accompanying the temperature rise. FIG. 6A shows a result of the reaction solution to which both the plasmid solution and the blood plasma-derived nucleic acid sample were added and FIG. 6B shows the result of a reaction solution to which only the plasmid solution was added. In each of the graphs shown in FIGs. 6A and 6B, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit. The Tm values determined beforehand are as follows.

With respect to the reaction solution to which the blood plasma-derived nucleic acid sample was added shown in FIG. 6A, the peaks were detected at around 73°C and around 48°C. With respect to the reaction solution to which only the plasmid solution was added shown in FIG. 6B, the peak was detected at around 48°C. These results show that peaks can be set at different Tm values by changing the complementarity to the probe even when the same probe 1 is used.

### Example A2

### Example A2-1

PCR and Tm analysis were performed with respect to a reaction system containing a control template in addition to a reaction system containing a specimen nucleic acid, and the amplification efficiency of the reaction systems was examined. In this Example, as described later, the probe and the control template were designed as the embodiment (Tm_{A} < Tm_{C}) shown in FIG. 5.

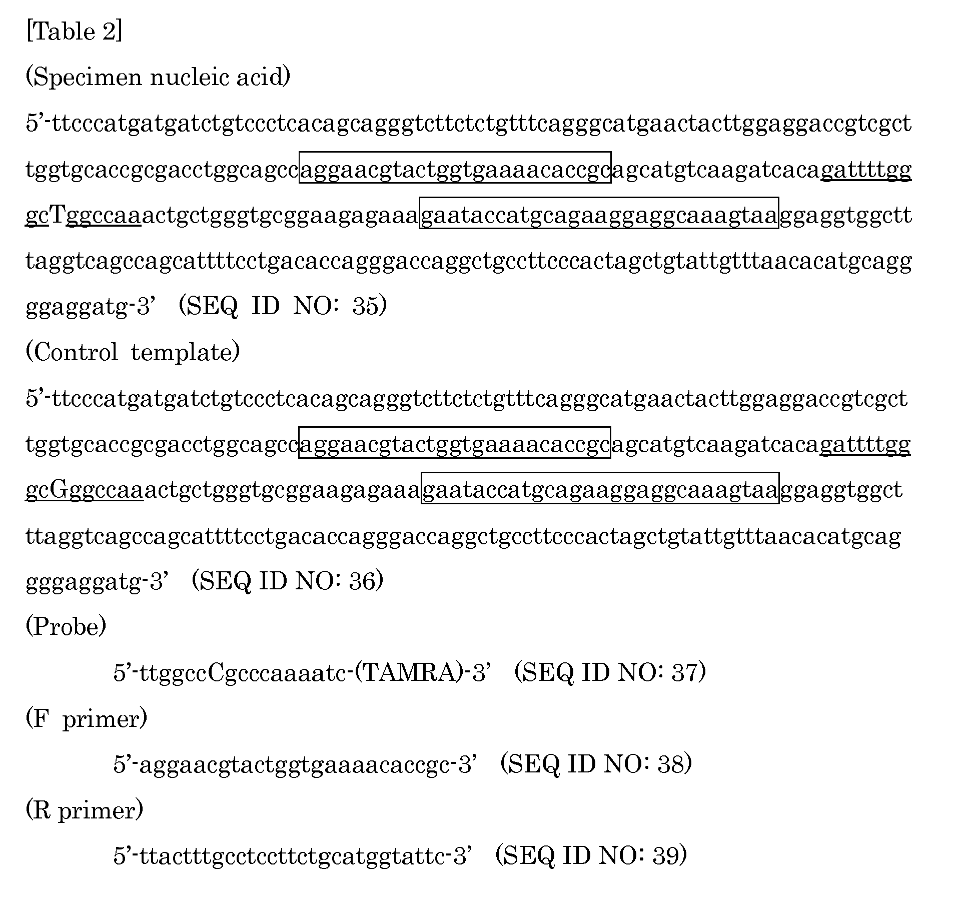

In the sequences of the specimen nucleic acid and the control template, the enclosed sequences are sequences complementary to F primer and R primer and the underlined sequences are sequences complementary to the probe. The probe and the control template were designed as the embodiment shown in FIG. 5. That is, the probe has a sequence that is perfectly complementary to the control template and that is partially non-complementary to the specimen nucleic acid. In this state, the Tm value (Tm_{A}) of a double-stranded nucleic acid composed of the amplification region of the specimen nucleic acid and the probe obtained beforehand was 53°C and the Tm value (Tm_{C}) of a double-stranded nucleic acid composed of the amplification region of the control template and the probe obtained beforehand was 64°C.

**[Table 3]**

| (First reagent: µL) | |
|---|---|
| Distilled water | 19.755 |
| 1 mol/L Tris-HCl (pH 8.6) | 0.63 |
| 20 w/v% BSA | 0.5 |
| 10 w/v% NaN₃ | 0.115 |
| 0.94 U/µL Taq polymerase | 2 |
| Total | 23 µL |
| | |

| (Second reagent: µL) | |
|---|---|
| Distilled water | 8.6225 |
| 10 w/v% NaN₃ | 0.065 |
| 1 mol/L Tris-HCl (pH 8.6) | 0.35 |
| 25 mmol/L dNTP | 0.4 |
| 80 v/v% glycerol | 1.56 |
| 100 mmol/L MgCl₂ | 0.75 |
| 1 mol/L KCl | 1.25 |
| Total | 13 µL |
| | |

| (Third reagent: µL) | |
|---|---|
| Distilled water | 8.83 |
| 1 mol/L Tris-HCl (pH 8.6) | 0.27 |
| 10 w/v% NaN₃ | 0.05 |
| 100 µmol/L probe | 0.1 |
| 100 µmol/L F primer | 0.5 |
| 100 µmol/L R primer | 0.25 |
| Total | 10 µL |

1 µL of the specimen nucleic acid or 1µL of the control template nucleic acid was mixed with 23 µL of the first reagent, 13 µL of the second reagent, 10 µL of the third reagent, and 3 µL of distilled water, and then 30 µL of mineral oil (product name: Mineral oil, produced by Nacalai Tesque, Inc.) was added thereto to prepare a reaction solution. A reaction solution containing the specimen nucleic acid was used as a specimen reaction solution. A reaction solution containing the control template nucleic acid was used as a control reaction solution. PCR was performed with reference to each of the reaction solutions using a fully-automated SNP analyzer (product name: i-densy IS-5310, produced by ARKRAY, Inc.). Conditions for PCR were as follows. That is, after treating at 95°C for 60 seconds, one cycle of treatment at 95°C for 1 second and at 58°C for 15 seconds was repeated for 50 cycles. Further, after treating at 95°C for 1 second and at 40°C for 60 seconds, the temperature was raised from 40°C to 85°C at a temperature rise rate of 1°C/3 sec. During the temperature rise, the change in fluorescence intensity at each temperature was measured and the Tm analysis was performed. It is to be noted that the detection of the fluorescence intensity was carried out at the wavelength of 585 nm to 700 nm (for detection of the fluorescent substance, TAMRA).

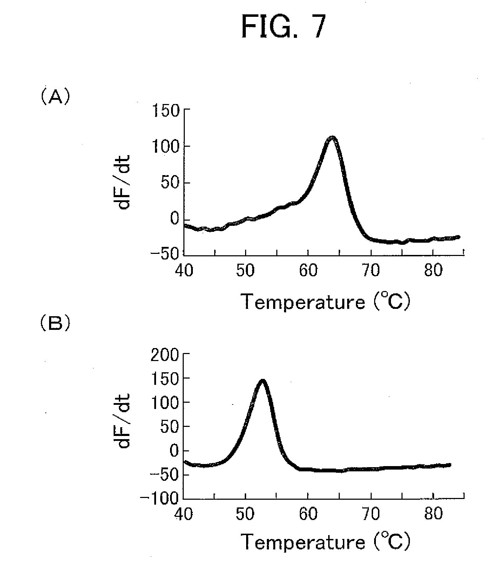

The results thereof are shown in FIGs. 7A and 7B. FIGs. 7A and 7B show graphs of the Tm analysis indicating the change in fluorescence intensity accompanying the temperature rise. In each of the graphs shown in FIGs. 7A and 7B, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit. FIG. 7A shows the result of the reaction solution containing the control template and FIG. 7B shows the result of the reaction solution containing the specimen nucleic acid.

As shown in FIG. 7A, in the reaction solution containing the control template, the peak was detected only at around the Tm value (Tm_{C}) 64°C of a double-stranded nucleic acid composed of the control template and the probe. As shown in FIG. 7B, in the reaction solution containing the specimen nucleic acid, the peak was detected only at around the Tm value (Tm_{A}) 53°C of a double-stranded nucleic acid composed of the specimen nucleic acid and the probe. These results tell the following points. First, in the control reaction solution, the occurrence of the peak at the Tm value (Tm_{C}) of a double-stranded nucleic acid composed of the control template and the probe shows that the control template is amplified normally. Since the peak occurs at around the Tm value 64°C in FIG. 7A, it was proved that the reaction solution containing the control template was positive control (+), i.e., it was proved that a normal amplification reaction occurred in the reaction solution containing the control template. Further, the composition of the reaction solution used for the amplification of the control template is the same as that of the reaction solution used for the amplification of the specimen nucleic acid except that it contains the control template nucleic acid instead of the specimen nucleic acid. The reaction conditions for the control reaction solution are the same as those for the specimen reaction solution. From these, it was indirectly proved that the occurrence of the peak in the specimen reaction solution was caused by the normal amplification. Further, in the control reaction solution, the occurrence of the peak at a temperature other than the Tm value (Tm_{C}) of a double-stranded nucleic acid composed of the control template and the probe shows that the reaction solution was contaminated with undesired nucleic acid and that amplification of the undesired nucleic acid occurred. Since the peak does not occur at a temperature other than the Tm value (Tm_{C}) 64°C of a double-stranded nucleic acid composed of the control template and the probe in FIG. 7A, it was proved that the control reaction solution was negative control (-), i.e., it was proved that the reaction solution was not contaminated with undesired nucleic acid and that the amplification of the undesired nucleic acid did not occur. Further, the specimen reaction solution was prepared in the same manner as the control reaction solution to carry out the reaction. From these, it was indirectly proved that the peak obtained at around the Tm value (Tm_{A}) 53°C of a double-stranded nucleic acid composed of the specimen nucleic acid and the probe in the specimen reaction solution was not caused by the amplification of the contaminating undesired nucleic acid but caused by the amplification of the specimen nucleic acid.

### Example A2-2

With respect to a reaction solution contaminated with undesired nucleic acid, determination of a negative control was made using a control template. It is to be noted that Example A2-2 was performed in the same manner as in Example A2-1 unless otherwise stated.

0.1 µL of the control template of Example A2-1, 23 µL of the first reagent, 13 µL of the second reagent, 10 µL of the third reagent, and 3 µL of distilled water were mixed. Thereafter, 0.9 µL of the following contaminating nucleic acid was mixed therein and 30 µL of the mineral oil was further added to prepare a control reaction solution. Then, PCR and Tm analysis were performed in the same manner as in Example A2-1. The following contaminating nucleic acid has a sequence to which each primer can be annealed and the probe of Example A2-1 can be hybridized. In the following sequence, the enclosed sequences are sequences complementary to F primer and R primer and the underlined parts indicate sequences complementary to the probe. In this state, the Tm value (Tm_{C}) of a double-stranded nucleic acid composed of the amplification region of the control template and the probe obtained beforehand was 64°C and the Tm value of a double-stranded nucleic acid composed of the amplification region of the contaminating nucleic acid and the probe obtained beforehand was 53°C.

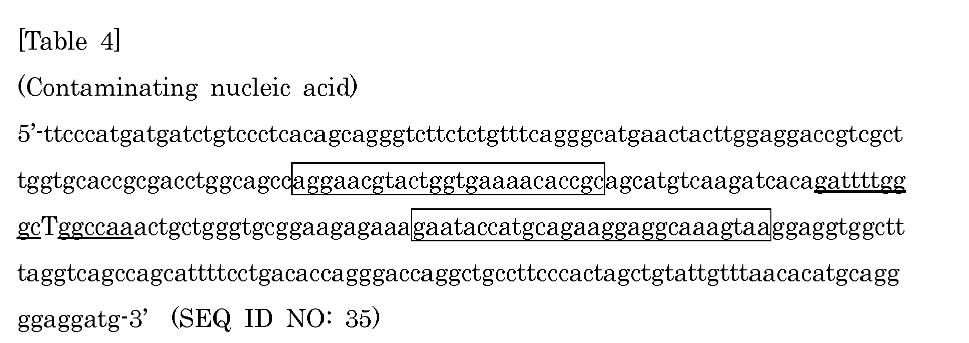

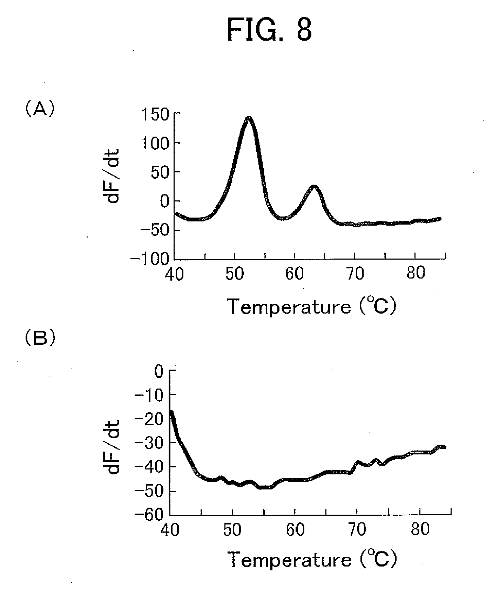

The results thereof are shown in FIG. 8A. FIG. 8A shows a graph of the Tm analysis indicating the change in fluorescence intensity accompanying the temperature rise with respect to the control reaction solution. In FIG. 8A, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit.

As shown in FIG. 8A, in the control reaction solution, the peaks were detected both at around the Tm value (Tm_{C}) 64°C of a double-stranded nucleic acid composed of the control template and the probe and at around the Tm value 53°C of a double-stranded nucleic acid composed of the contaminating nucleic acid and the probe. Since the peak occurred at around the former Tm value (Tm_{C}) 64°C, it was proved that the reaction solution containing the control template was positive control (+), i.e., it was proved that a normal amplification reaction occurred in the reaction solution containing the control template. However, since the peak also occurred at around the latter Tm value 53°C, it was proved that the reaction solution containing the control template was negative control (+), i.e., it was proved that the reaction solution was contaminated with undesired nucleic acid and the amplification of the undesired nucleic acid occurred. In this manner, amplification due to contaminating undesired nucleic acid can be determined on the basis of whether or not a peak is present at a temperature other than the Tm value of a double-stranded nucleic acid composed of a control template and a probe in a control reaction solution.

### Example A2-3

With respect to a reaction solution to which SDS that inhibits an amplification reaction was added, determination of a positive control was made using a control template. It is to be noted that Example A2-3 was performed in the same manner as in Example A2-2 unless otherwise stated.

PCR and Tm analysis were performed in the same manner as in Example A2-2 except that 3 µL of 10 v/v% SDS instead of 3 µL of distilled water was added to the control reaction system of Example A2-2.

The results thereof are shown in FIG. 8B. FIG. 8B shows a graph of the Tm analysis showing the change in fluorescence intensity accompanying the temperature rise with respect to the control reaction solution. In FIG. 8B, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit.

As shown in FIG. 8B, in the control reaction solution, the peak was not detected at any temperature. From these data, it was proved that a normal amplification reaction did not occur. In this manner, it can be determined whether or not a normal amplification reaction occurred on the basis of the presence or absence of a peak at the Tm value of a double-stranded nucleic acid composed of a control template and a probe.

### Example A3

### Example A3-1

PCR and Tm analysis were performed with respect to a reaction system containing a control template in addition to a reaction system containing a specimen nucleic acid, and the amplification efficiency of the reaction systems was examined. In this Example, as described later, the probe and the control template were designed as the embodiment (Tm_{A} > Tm_{C}) shown in FIG. 4.

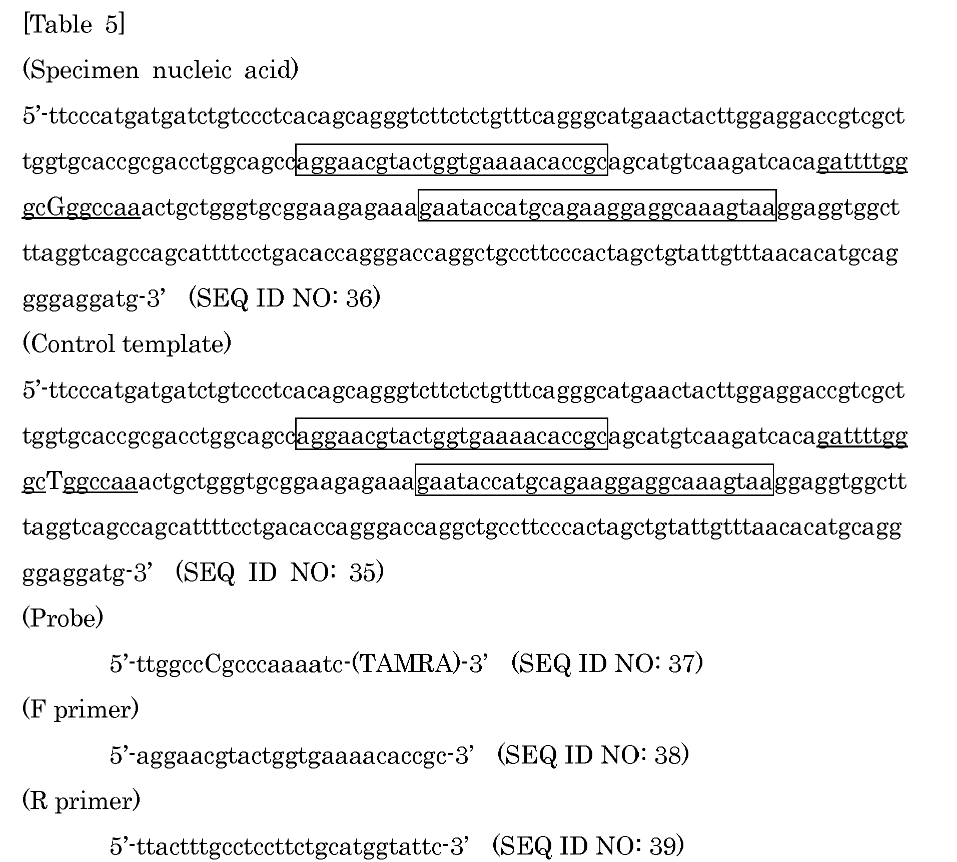

In the sequences of the specimen nucleic acid and the control template, the enclosed sequences are sequences complementary to F primer and R primer and the underlined sequences are sequences complementary to the probe. The probe and the control template were designed as the embodiment shown in FIG. 4. That is, the probe has a sequence that is perfectly complementary to the specimen nucleic acid and that is partially non-complementary to the control template. In this state, the Tm value (Tm_{A}) of a double-stranded nucleic acid composed of the amplification region of the specimen nucleic acid and the probe obtained beforehand was 64°C and the Tm value (Tm_{C}) of a double-stranded nucleic acid composed of the amplification region of the control template and the probe obtained beforehand was 53°C.

PCR and Tm analysis were performed in the same manner as in Example A2-1 except that 1 µL of the specimen nucleic acid or 1 µL of the control template nucleic acid was used.

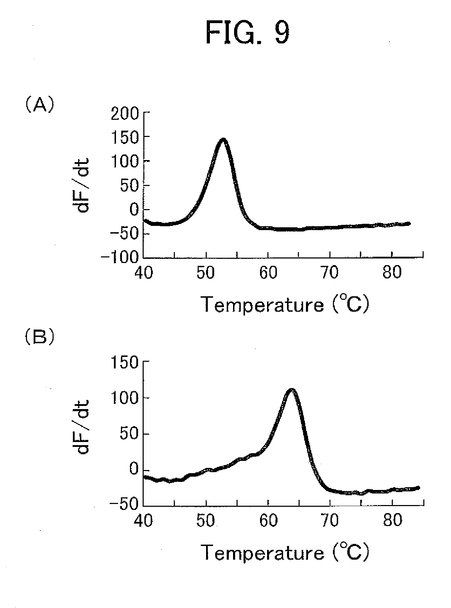

The results thereof are shown in FIGs. 9A and 9B. FIGs. 9A and 9B show graphs of the Tm analysis indicating the change in fluorescence intensity accompanying the temperature rise. In each of the graphs shown in FIGs. 9A and 9B, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit. FIG. 9A shows the result of the reaction solution containing the control template and FIG. 9B shows the result of the reaction solution containing the specimen nucleic acid.

As shown in FIG. 9A, in the reaction solution containing the control template, the peak was detected only at around the Tm value (Tm_{C}) 53°C of a double-stranded nucleic acid composed of the control template and the probe. As shown in FIG. 9B, in the reaction solution containing the specimen nucleic acid, the peak was detected only at around the Tm value (Tm_{A}) 64°C of a double-stranded nucleic acid composed of the specimen nucleic acid and the probe. In this manner, since the peak was detected only at around the Tm value (Tm_{C}) 53°C of a double-stranded nucleic acid composed of the control template and the probe, it was proved that the control reaction solution was positive control (+) and negative control (-) as in the case of Example A2-1. From these, it was indirectly proved that the occurrence of the peak in the specimen reaction solution was caused by a normal amplification. Further, the peak obtained at around the Tm value (Tm_{A}) 64°C of a double-stranded nucleic acid composed of the specimen nucleic acid and the probe was not caused by the amplification of the contaminating undesired nucleic acid but caused by the amplification of the specimen nucleic acid.

### Example A3-2

With respect to a reaction solution contaminated with undesired nucleic acid, determination of a negative control was made using a control template. It is to be noted that Example A3-2 was performed in the same manner as in Example A3-1 unless otherwise stated.

0.9 µL of the control template of Example A3-1, 23 µL of the first reagent, 13 µL of the second reagent, 10 µL of the third reagent, and 3 µL of distilled water were mixed. Thereafter, 0.1 µL of the following contaminating nucleic acid was mixed therein and 30 µL of the mineral oil was further added to prepare a control reaction solution. Then, PCR and Tm analysis were performed in the same manner as in Example A3-1. The following contaminating nucleic acid has a sequence to which each primer can be annealed and the probe of Example A3-1 can be hybridized. In the following sequence, the enclosed sequences are sequences complementary to F primer and R primer and the underlined sequence is a sequence complementary to the probe. In this state, the Tm value (Tm_{C}) of a double-stranded nucleic acid composed of the amplification region of the control template and the probe obtained beforehand was 53°C and the Tm value of a double-stranded nucleic acid composed of the amplification region of the contaminating nucleic acid and the probe obtained beforehand was 64°C.

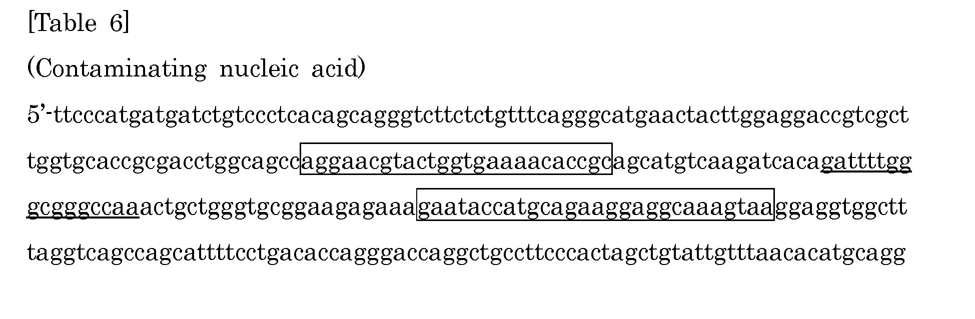

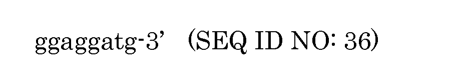

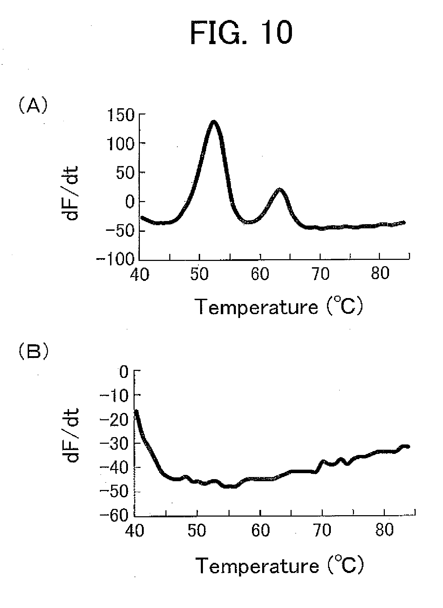

The results thereof are shown in FIG. 10A. FIG. 10A shows a graph of the Tm analysis indicating the change in fluorescence intensity accompanying the temperature rise with respect to the control reaction solution. In FIG. 10A, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit.

As shown in FIG. 10A, in the control reaction solution, the peaks were detected both at around the Tm value (Tm_{C}) 53°C of a double-stranded nucleic acid composed of the control template and the probe and at around the Tm value 64°C of a double-stranded nucleic acid composed of the contaminating nucleic acid and the probe. Since the peak occurred at around the former Tm value 53°C, it was proved that the reaction solution containing the control template was positive control (+), i.e., it was proved that a normal amplification reaction occurred in the reaction solution containing the control template. However, since the peak also occurred at around the latter Tm value 64°C, it was proved that the reaction solution containing the control template was negative control (+), i.e., it was proved that the reaction solution was contaminated with undesired nucleic acid and the amplification of the undesired nucleic acid occurred. In this manner, amplification due to contaminating undesired nucleic acid can be determined on the basis of whether or not a peak is present at a temperature other than the Tm value of a double-stranded nucleic acid composed of a control template and a probe in the control reaction solution.

### Example A3-3

With respect to a reaction solution to which SDS that inhibits an amplification reaction was added, determination of a positive control was made using a control template. It is to be noted that Example A3-3 was performed in the same manner as in Example A3-2 unless otherwise stated.

PCR and Tm analysis were performed in the same manner as in Example A3-2 except that 3 µL of 10 v/v% SDS instead of 3 µL of distilled water was added to the control reaction system of Example A3-2.

The results thereof are shown in FIG. 10B. FIG. 10B shows a graph of the Tm analysis showing the change in fluorescence intensity accompanying the temperature rise with respect to the control reaction solution. In FIG. 10B, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit.

As shown in FIG. 10B, in the control reaction solution, the peak was not detected at any temperature. From these, it was proved that a normal amplification reaction did not occur. In this manner, it can be determined whether or not a normal amplification reaction occurred on the basis of the presence or absence of a peak at the Tm value of a double-stranded nucleic acid composed of a control template and a probe.

### Example A4

### Example A4-1

PCR and Tm analysis were performed with respect to a reaction system containing a control template in addition to a reaction system containing a specimen nucleic acid, and the amplification efficiency of the reaction systems was examined. In this Example, as described later, the probe and the control template were designed as the embodiment (Tm_{A} < Tm_{C}) shown in FIG. 5.

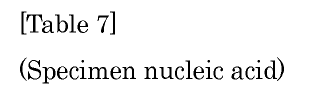

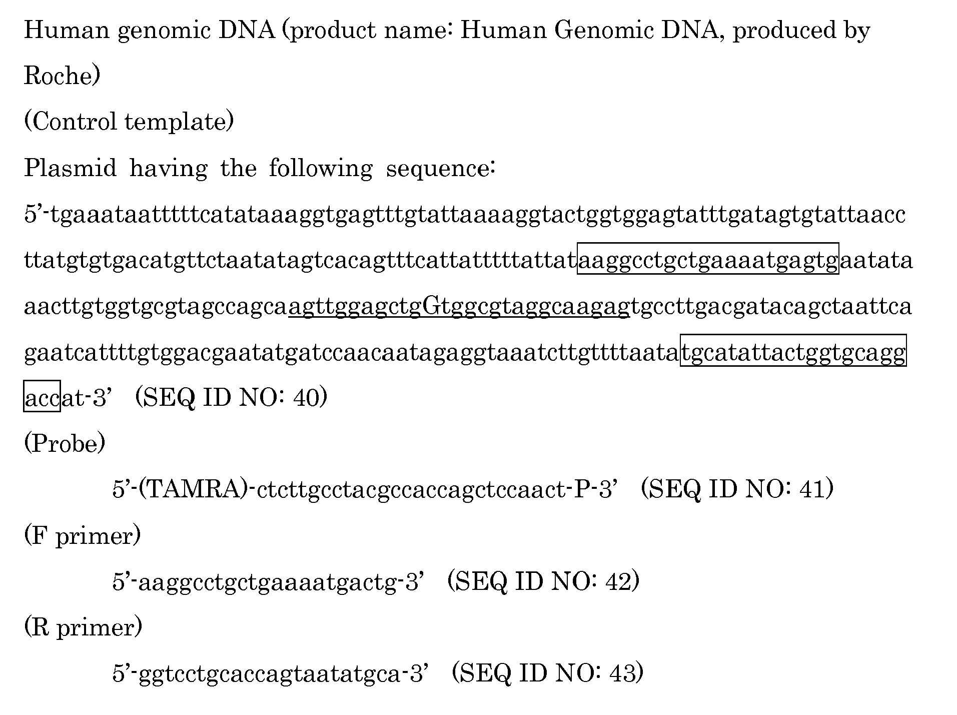

In the sequences of the specimen nucleic acid and the control template, the enclosed sequences are sequences complementary to F primer and R primer and the underlined sequence is a sequence complementary to the probe. The probe and the control template were designed as the embodiment shown in FIG. 5. That is, the probe has a sequence that is perfectly complementary to the control template and that is partially non-complementary to the specimen nucleic acid. In this state, the Tm value (Tm_{A}) of a double-stranded nucleic acid composed of the amplification region of the specimen nucleic acid and the probe obtained beforehand was 71°C and the Tm value (Tm_{C}) of a double-stranded nucleic acid composed of the amplification region of the control template and the probe obtained beforehand was 79°C.

**[Table 8]**

| (Fourth reagent: µL) | |
|---|---|
| Distilled water | 5.93 |
| 1 mol/L Tris-HCl (pH 8.6) | 0.27 |
| 10 w/v% NaN₃ | 0.05 |
| 100 µmol/L probe | 2 |
| 100 µmol/L F primer | 0.5 |
| 100 µmol/L R primer | 0.25 |
| Total | 9 µL |

1 µL of the control template (10⁶ copies/µL), 23 µL of the first reagent, 13 µL of the second reagent, 9 µL of the fourth reagent, and 4 µL of distilled water were mixed, and then 30 µL of the mineral oil was further added thereto to prepare a reaction solution. A reaction solution containing the control template was used as a control reaction solution. Further, 1 µL of the specimen nucleic acid (30 copies/µL), 23 µL of the first reagent, 13 µL of the second reagent, 9 µL of the fourth reagent, and 4 µL of distilled water were mixed, and then 30 µL of the mineral oil was further added thereto to prepare a reaction solution. A reaction solution containing the specimen nucleic acid was used as a specimen reaction solution. PCR was performed with reference to each of the reaction solutions using a fully-automated SNP analyzer (product name: i-densy IS-5310, produced by ARKRAY, Inc.). Conditions for PCR were as follows. That is, after treating at 95°C for 60 seconds, one cycle of treatment at 95°C for 1 second and at 60°C for 15 seconds was repeated for 50 cycles. Further, after treating at 95°C for 1 second and at 55°C for 60 seconds, the temperature was raised from 62°C to 85°C at a temperature rise rate of 1°C/3 sec. During the temperature rise, the change in fluorescence intensity at each temperature was measured and the Tm analysis was performed. It is to be noted that the detection of the fluorescence intensity was carried out at the wavelength of 585 nm to 700 nm (for detection of the fluorescent substance, TAMRA).

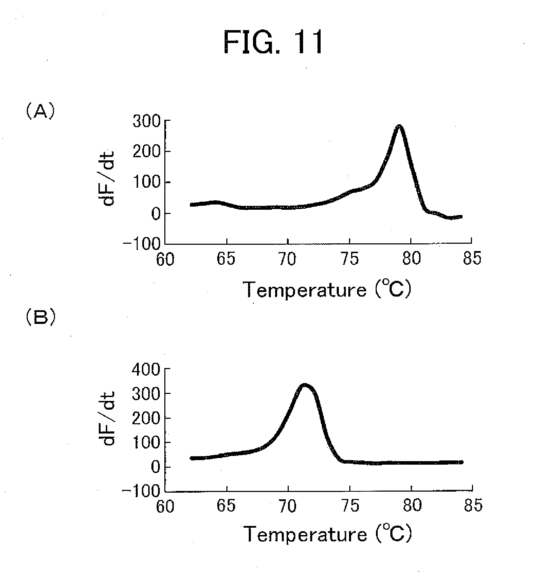

The results thereof are shown in FIGs. 11A and 11B. FIGs. 11A and 11B shows graphs of the Tm analysis indicating the change in fluorescence intensity accompanying the temperature rise. In each of the graphs shown in FIGs. 11A and 11B, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit. FIG. 11A shows the result of the reaction solution containing the control template and FIG. 11B shows the result of the reaction solution containing the specimen nucleic acid.

As shown in FIG. 11A, in the reaction solution containing the control template, the peak was detected only at around the Tm value (Tm_{C}) 79°C of a double-stranded nucleic acid composed of the control template and the probe. As shown in FIG. 11B, in the reaction solution containing the specimen nucleic acid, the peak was detected only at around the Tm value (Tm_{A}) 71°C of a double-stranded nucleic acid composed of the specimen nucleic acid and the probe. In this manner, since the peak was detected only at around the Tm value (Tm_{C}) 79°C of a double-stranded nucleic acid composed of the control template and the probe, it was proved that the control reaction solution was positive control (+) and negative control (-) as in the case of Example A2-1. From these, it was indirectly proved that the occurrence of the peak in the specimen reaction solution was caused by a normal amplification. Further, the peak obtained at around the Tm value (Tm_{A}) 71°C of a double-stranded nucleic acid composed of the specimen nucleic acid and the probe was not caused by the amplification of the contaminating undesired nucleic acid but caused by the amplification of the specimen nucleic acid.

### Example A4-2

With respect to a reaction solution contaminated with undesired nucleic acid, determination of a negative control was made using a control template. It is to be noted that Example A4-2 was performed in the same manner as in Example A4-1 unless otherwise stated.

1 µL of the control template of Example A4-1, 23 µL of the first reagent, 13 µL of the second reagent, 9 µL of the fourth reagent, and 3 µL of distilled water were mixed. Thereafter, 1 µL of a plasmid (30 copies/µL) having the following sequence as a contaminating nucleic acid was mixed therein and 30 µL of the mineral oil was further added thereto to prepare a control reaction solution. Then, PCR and the Tm analysis were performed in the same manner as in Example A4-1. The following contaminating nucleic acid has a sequence to which each primer can be annealed and the probe of Example A4-1 can be hybridized. In the following sequence, the enclosed sequences are sequences complementary to F primer and R primer and the underlined sequences are sequences complementary to the probe. In this state, the Tm value (Tm_{C}) of a double-stranded nucleic acid composed of the amplification region of the control template and the probe obtained beforehand was 79°C and the Tm value of a double-stranded nucleic acid composed of the amplification region of the contaminating nucleic acid and the probe obtained beforehand was 65°C.

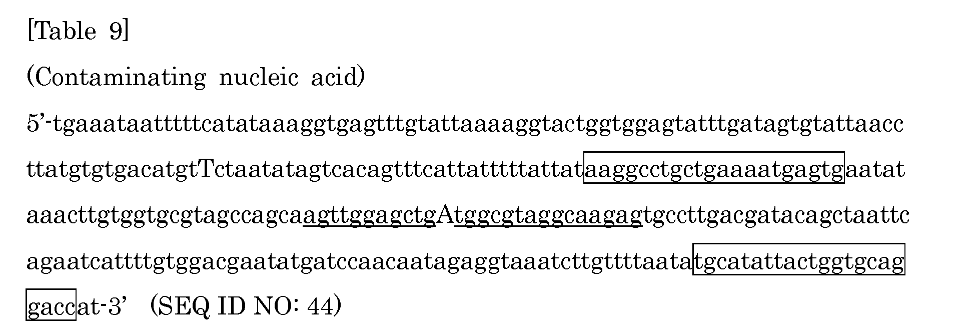

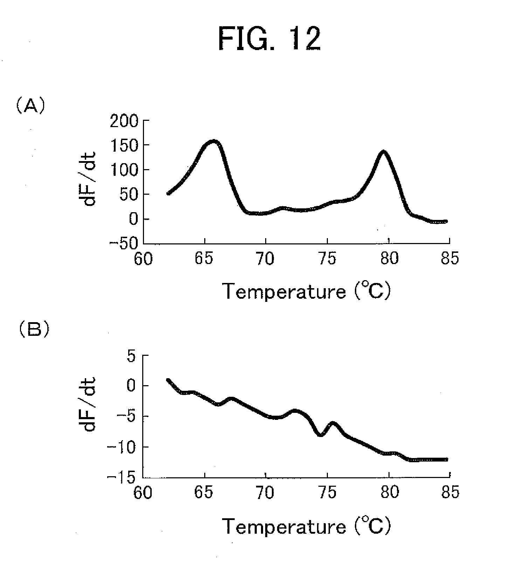

The results thereof are shown in FIG. 12A. FIG. 12A shows a graph of the Tm analysis indicating the change in fluorescence intensity accompanying the temperature rise with respect to the control reaction solution. In FIG. 12A, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit.

As shown in FIG. 12A, in the control reaction solution, the peaks were detected both at around the Tm value (Tm_{C}) 79°C of a double-stranded nucleic acid composed of the control template and the probe and at around the Tm value 65°C of a double-stranded nucleic acid composed of the contaminating nucleic acid and the probe. Since the peak occurred at around the former Tm value 79°C, it was proved that the reaction solution containing the control template was positive control (+), i.e., it was proved that a normal amplification reaction occurred in the reaction solution containing the control template. However, since the peak also occurred at around the latter Tm value 65°C, it was proved that the reaction solution containing the control template was negative control (+), i.e., it was proved that the reaction solution was contaminated with undesired nucleic acid and the amplification of the undesired nucleic acid occurred. In this manner, amplification due to contaminating undesired nucleic acid can be determined on the basis of whether or not the peak is present at a temperature other than the Tm value of a double-stranded nucleic acid composed of the control template and the probe in the control reaction solution.

### Example A4-3

With respect to a reaction solution to which SDS that inhibits an amplification reaction was added, determination of a positive control was made using a control template. It is to be noted that Example A4-3 was performed in the same manner as in Example A4-2 unless otherwise stated.

PCR and Tm analysis were performed in the same manner as in Example A4-2 except that a reaction solution was prepared by mixing 1 µL of the control template of Example A4-1, 23 µL of the first reagent, 13 µL of the third reagent, 9 µL of the fourth reagent, and 4 µL of human genomic DNA (product name: Human Genomic DNA, produced by Roche) as a specimen nucleic acid and 1 µL of SDS. It is to be noted that the final concentration of the human genomic DNA in the reaction solution was 10³ copies/µL.

The results thereof are shown in FIG. 12B. FIG. 12B shows a graph of the Tm analysis showing the change in fluorescence intensity accompanying the temperature rise with respect to the control reaction solution. In FIG. 12B, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit.

As shown in FIG. 12B, in the control reaction solution, the peak was not detected at any temperature. From these, it was proved that a normal amplification reaction did not occur. In this manner, it can be determined whether or not a normal amplification reaction occurred on the basis of the presence or absence of a peak at the Tm value of a double-stranded nucleic acid composed of a control template and a probe.

### Example B1

In this example, it was examined whether or not the temperature control and the signal detection were carried out normally using the control oligonucleotide and the control probe. In addition, polymorphisms (CYP2C19*2 and CYP2C19*3) in the target site of the CYP2C19 gene were detected with respect to various nucleic acid samples by a Tm analysis. CYP2C19*2 is a polymorphism at the position 681 in the exon 5 of the CYP2C19 gene. The wild-type thereof is guanine and the mutant-type thereof is adenine. Further, CYP2C19*3 is a polymorphism at the position 636 in the exon 4 of the CYP2C19 gene. The wild-type thereof is guanine and the mutant-type thereof is adenine.

**[Table 10]**

| (PCR reaction solution composition: µL) | |
|---|---|
| Distilled water | 32.4 |
| 80 v/v% Glycerol | 5 |
| 1 mol/L Tris-HCl (pH 8.6) | 1.25 |
| 1 mol/L KCl | 1.25 |
| 10 mmol/L dNTP | 1 |
| 1 mol/L MgCl₂ | 0.225 |
| 20 w/v% BSA | 0.5 |
| 10 w/v% NaN₃ | 0.23 |
| 100 µmol/L CYP2C19*2 F primer | 0.25 |
| 100 µmol/L CYP2C19*2 R primer | 0.5 |
| 5 µmol/L CYP2C19*2 probe | 0.2 |
| 100 µmol/L CYP2C19*3 F primer | 0.25 |
| 100 µmol/L CYP2C19*3 R primer | 0.5 |
| 5 µmol/L CYP2C19*3 probe | 0.2 |
| 100 µmol/L Tm control oligonucleotide | 0.1 |
| 100 µmol/L Tm control probe | 0.1 |
| 0.94 U/µL Taq polymerase | 2 |
| Total | 46 µL |

(CYP2C19*2)
F primer (SEQ ID NO: 11)
   5'-taaattattgttttctcttagatatgcaataattttccca-3'
R primer (SEQ ID NO: 12)
   5'-cccgagggttgttgatgtccatc-3'
Probe (SEQ ID NO: 13)
   5'-tcccaggaacccataac-(BODIPY FL)-3'
(CYP2C19*3)
F primer (SEQ ID NO: 14)
   5'-tgatggaaaaattgaatgaaaacatcaggattgta-3'
R primer (SEQ ID NO: 15)
   5'-tcaaaaatgtacttcagggcttggtcaata-3'
Probe (SEQ ID NO: 16)
   5'-(TAMRA)-ccctgaatccaggtaag-P-3'
(Tm control reagent)
Tm control oligonucleotide (SEQ ID NO: 17)
   5'-gcatggcttccattggg-P-3'
Tm control probe (SEQ ID NO: 18)
   5'-(Pacific Blue)-cccaatggaagccatgc-P-3'

### (Diluent 1)

10 mmol/L Tris-HCl (pH8)
0.1 mmol/L EDTA
0.3 v/v% SDS
0.05 w/v% sodium azide

### (Diluent 2)

10 mmol/L Tris-HCl (pH8)
0.1 mmol/L EDTA
0.05 w/v% sodium azide

### (Purified Human Genome)

A purified human genome sample was prepared using a nucleic acid purification kit (product name: Q1Aamp, produced by Qiagen). It was preliminarily confirmed that the polymorphisms, CYP2C19*2 and CYP2C19*3, of this purified human genome sample were both heterozygotes (wild/mutant). 1 µL of the purified human genome sample (0.3 ng/µL) was added to 46 µL of the PCR reaction solution, and PCR was performed using a fully-automated SNP analyzer (product name: i-densy LS-5310, produced by ARKRAY, Inc.). Conditions for PCR were as follows. That is, after treating at 90°C for 60 seconds, one cycle of treatment at 95°C for 1 second and at 64°C for 15 seconds was repeated for 50 cycles. Further, after treating at 95°C for 1 second and at 40°C for 60 seconds, the temperature was raised from 40°C to 75°C at a temperature rise rate of 1°C/3 sec. During the temperature rise, the change in fluorescence intensity at each temperature was measured and the Tm analysis was performed. It is to be noted that the detection of the fluorescence intensity was carried out at the wavelength of 450 nm to 480 nm (for detection of the fluorescent substance, Pacific Blue), at the wavelength of 515 nm to 555 nm (for detection of the fluorescent substance, BODIPY FL), and at the wavelength of 585 nm to 700 nm (for detection of the fluorescent substance, TAMRA).

### (Plasmid)

As partial sequences of the CYP2C19 gene, a wild-type plasmid (WT*2) in which the oligonucleotide (SEQ ID NO: 27) of the wild-type CYP2C19*2 was inserted, a wild-type plasmid (WT*3) in which the oligonucleotide (SEQ ID NO: 29) of the wild-type CYP2C19*3 was inserted, a mutant-type plasmid (mt*2) in which the oligonucleotide (SEQ ID NO: 28) of the mutant-type CYP2C19*2 was inserted, and a mutant-type plasmid (mt*3) in which the oligonucleotide (SEQ ID NO: 30) of the mutant-type CYP2C19*3 was inserted were prepared. Next, a plasmid sample (WT) was prepared by mixing the wild-type plasmid (WT*2) and the wild-type plasmid (WT*3) so that the concentration of each plasmid becomes 400 copies/µL. Further, a plasmid sample (mt) was prepared by mixing the mutant-type plasmid (mt*2) and the mutant-type plasmid (mt*3) so that the concentration of each plasmid becomes 400 copies/µL. Then, 10 µL of the plasmid sample and 70 µL of the diluent 1 were mixed and 10 µL of this mixture was further mixed with 70 µL of the diluent 2. Thereafter, 17 µL of the resultant mixture was treated at 95°C for 10 minutes and added to 46 µL of the PCR reaction solution, and then PCR and Tm analysis were performed in the same manner as the purified human genome.

### (Blood)

Blood was collected using a EDTA blood collection tube. It was preliminarily confirmed that the polymorphisms, CYP2C19*2 and CYP2C19*3, of this blood sample were both heterozygotes (wild/mutant). 10 µL of the collected blood and 70 µL of the diluent 1 were mixed and 10 µL of this mixture was further mixed with 70 µL of the diluent 2. Thereafter, 17 µL of the resultant mixture was treated at 95°C for 10 minutes and added to 46 µL of the PCR reaction solution, and then PCR and Tm analysis were performed in the same manner as the purified human genome.

### (Control)

17 µL of the diluent 2 was treated at 95°C for 10 minutes and added to 46 µL of the PCR reaction solution, and then PCR and Tm analysis were performed in the same manner as the purified human genome.

The Tm value of a double-stranded nucleic acid composed of the Tm control oligonucleotide and the Tm control probe is 62°C. Therefore, when the peak is detected at 62°C in the Tm analysis, it can be determined that a temperature control unit and a signal detecting unit of a Tm analyzing device function normally. The Tm values under the normal temperature condition are as follows:
(i) Tm value of a double-stranded nucleic acid composed of a Tm control oligonucleotide and a Tm control probe: 62°C
(ii) Tm value of a double-stranded nucleic acid composed of a wild-type CYP2C19*2 and a CYP2C19*2 probe: 51°C
(iii) Tm value of a double-stranded nucleic acid composed of a mutant-type CYP2C19*2 and a CYP2C19*2 probe: 59°C
(iv) Tm value of a double-stranded nucleic acid composed of a wild-type CYP2C19*3 and a CYP2C19*3 probe: 50°C
(v) Tm value of a double-stranded nucleic acid composed of a mutant-type CYP2C19*3 and a CYP2C19*3 probe: 59°C

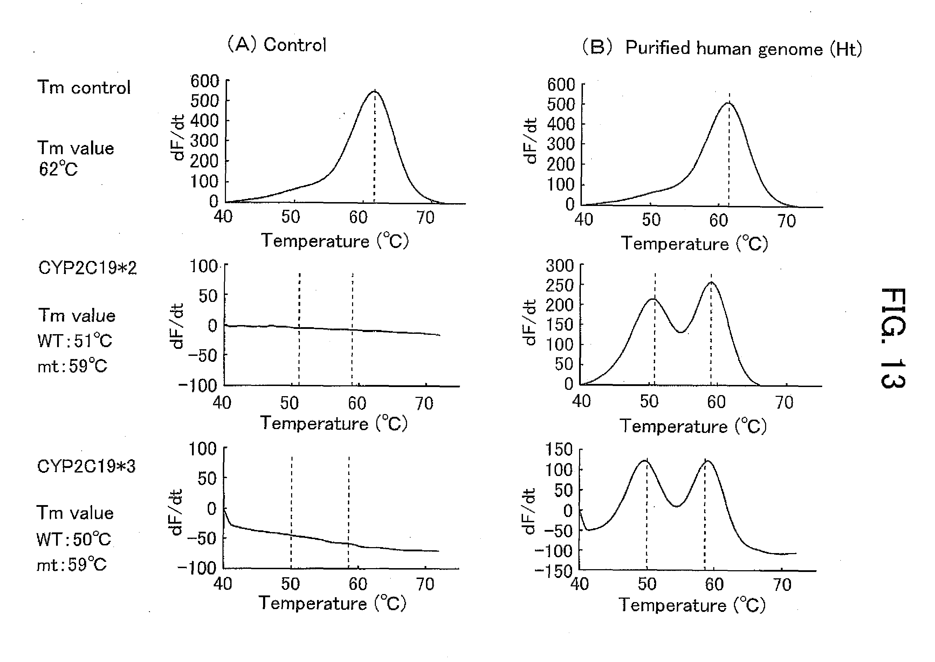

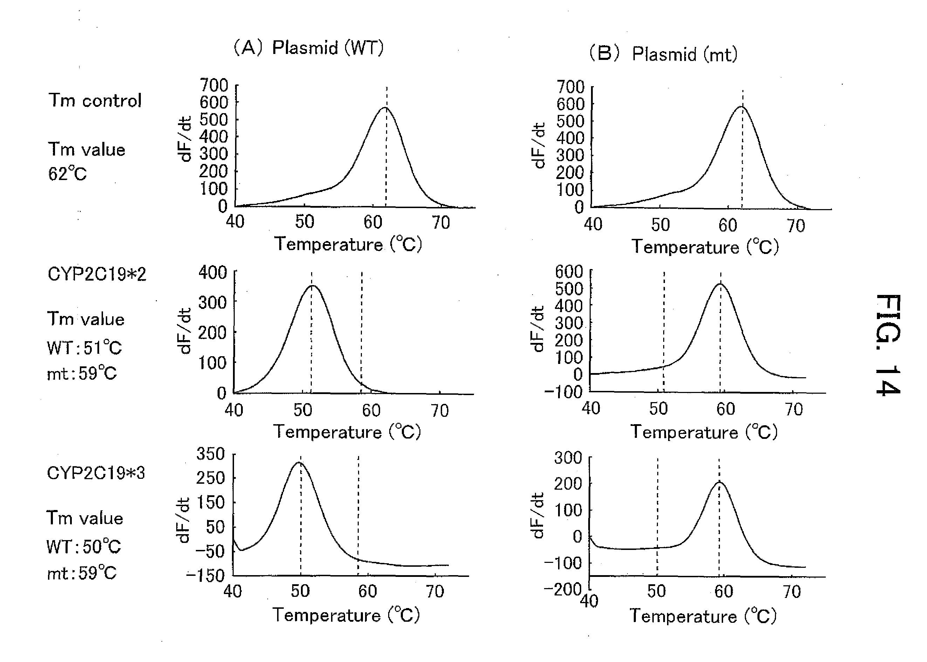

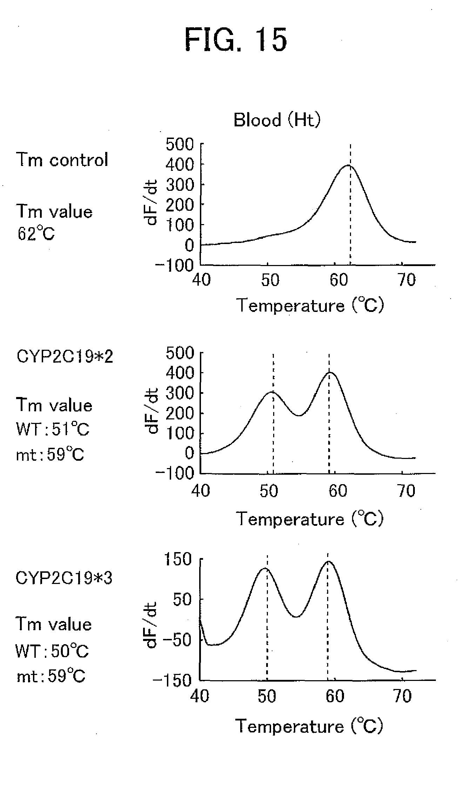

The results thereof are shown in FIGs. 13A to 15. FIGs. 13A to 15 show graphs of the Tm analysis indicating the change in fluorescence intensity accompanying the temperature rise. In each of the graphs shown in FIGs. 13A to 15, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit. FIG. 13A shows the result of the control, FIG. 13B shows the result of the purified human genome, FIG. 14A shows the result of the wild-type plasmid (WT), FIG. 14B shows the result of the mutant-type plasmid (mt), and FIG. 15 shows the result of the blood. In each of FIGs. 13A to 15, the graph (Tm control) on the upper side shows the fluorescence change on the basis of the Tm control probe, the graph (CYP2C19*2) on the middle shows the fluorescence change on the basis of the CYP2C19*2 probe, and the graph (CYP2C19*3) on the lower side shows the fluorescence change on the basis of the CYP2C19*3 probe.

As shown in FIG. 13A, in the control, the peak was detected only at around 62°C with respect to the Tm control probe and the peak was not detected with respect to the CYP2C19*2 probe and the CYP2C19*3 probe. As shown in FIG. 13B, the peak was detected at around 62°C with respect to the Tm control probe, the peaks were detected at around 51°C and 59°C with respect to the CYP2C19*2 probe, and the peaks were detected at around 50°C and 59°C with respect to the CYP2C19*3 probe. As shown in FIG. 14A, the peak was detected at around 62°C with respect to the Tm control probe, the peak was detected at around 51°C with respect to the CYP2C19*2 probe, and the peak was detected at around 50°C with respect to the CYP2C19*3 probe. As shown in FIG. 14B, the peak was detected at around 62°C with respect to the Tm control probe, the peak was detected at around 59°C with respect to the CYP2C19*2 probe, and the peak was detected at around 59°C with respect to the CYP2C19*3 probe. As shown in FIG. 15, the peak was detected at around 62°C with respect to the Tm control probe, the peaks were detected at around 51°C and 59°C with respect to the CYP2C19*2 probe, and the peaks were detected at around 50°C and 59°C with respect to the CYP2C19*3 probe. Since the peak was detected at around 62°C with respect to the Tm control probe in all the reaction solutions, it was confirmed that the temperature control of the Tm analyzing device was carried out normally. Further, although PCR and Tm analysis were performed in the presence of the Tm control oligonucleotide and the Tm control probe, the peak was not detected at a temperature other than the aforementioned Tm values with respect to the CYP2C19*2 probe and the CYP2C19*3 probe. This tells that even when the Tm control oligonucleotide and the Tm control probe are used together, it does not affect the detection of an objective polymorphism. In this manner, the present invention allows the detection of the polymorphism while examining whether or not the temperature control and the signal detection of the Tm analyzing device are carried out normally. Therefore, further reliable polymorphism analysis can be performed.

### Example B2

In this example, it was examined whether or not the temperature control and the signal detection were carried out normally using the control oligonucleotide and the control probe. In addition, polymorphisms (UGT1A1*6 and UGT1A1*28) in the target site of the UGT1A1 gene were detected with respect to various nucleic acid samples by a Tm analysis. UGT1A1*6 is a polymorphism at the position 211 in the exon 1 of the UGT1A1 gene. The wild-type thereof is guanine and the mutant-type thereof is adenine. Further, UGT1A1*28 is a polymorphism in a promoter region of the UGT1A1 gene, and is six TA repeats and seven TA repeats.

**[Table 11]**

| (PCR reaction solution composition: µL) | |
|---|---|
| Distilled water | 27.2 |
| 80 v/v% Glycerol | 9.375 |
| 1 mol/L Tris-HCl (pH 8.6) | 1.25 |
| 1 mol/L KCl | 2.5 |
| 10 mmol/L dNTP | 1 |
| 1 mol/L MgCl₂ | 0.075 |
| 20 w/v% BSA | 0.5 |
| 10 w/v% NaN₃ | 0.23 |
| 100 µmol/L UGT1A1*6 F primer | 0.25 |
| 100 µmol/L UGT1A1*6 R primer | 0.5 |
| 100 µmol/L UGT1A1*6 probe | 0.1 |
| 100 µmol/L UGT1A1*28 F primer | 0.25 |
| 100 µmol/L UGT1A1*28 R primer | 0.5 |
| 100 µmol/L UGT1A1*28 probe | 0.1 |
| 100 µmol/L Tm control oligonucleotide | 0.1 |
| 100 µmol/L Tm control probe | 0.1 |
| 0.94 U/µL Taq polymerase | 2 |
| Total | 46 µL |

(UGT1A1*6)
F primer (SEQ ID NO: 19)
   5'-tgaaatagttgtcctagcacctgacgc-3'
R primer (SEQ ID NO: 20)
   5'-caaaagactctttcacatcctccctttgg-3'
Probe (SEQ ID NO: 21)
   5'-gagacagagcattttacac-(TAMRA)-3'
(UGT1A1*28)
F primer (SEQ ID NO: 22)
   5'-agctttttatagtcacgtgacacagtcaaac-3'
R primer (SEQ ID NO: 23)
   5'-cgcctttgctcctgccagag-3'
Probe (SEQ ID NO: 24)
   5'-(BODIPY FL)-ccatatatatatatatataagtaggagag-P-3'
(Tm control reagent)
Tm control oligonucleotide (SEQ ID NO: 25)
   5'-gcatggcttccattggg-P-3'
Tm control probe (SEQ ID NO: 26)
   5'-(Pacific Blue)-cccaatggaagccatgc-P-3'

### (Purified Human Genome)

A purified human genome sample was prepared in the same manner as in Example B1. It was preliminarily confirmed that the polymorphisms, UGT1A1*28 and UGT1A1*6, of this purified human genome sample were both heterozygotes (wild/mutant). PCR and Tm analysis were performed in the same manner as the purified human genome in Example B1 except that this purified sample and the PCR reaction solution shown in Table 11 were used.

### (Plasmid)

As partial sequences of the UGT1A1 gene, a wild-type plasmid (WT*28) in which the oligonucleotide (SEQ ID NO: 31) of the wild-type UGT1A1*28 was inserted, a wild-type plasmid (WT*6) in which the oligonucleotide (SEQ ID NO: 33) of the wild-type UGT1A1*6 was inserted, a mutant-type plasmid (mt*28) in which the oligonucleotide (SEQ ID NO: 32) of the mutant-type UGT1A1*28 was inserted, and a mutant-type plasmid (mt*6) in which the oligonucleotide (SEQ ID NO: 34) of the mutant-type UGT1A1*6 was inserted were prepared. Next, a plasmid sample (WT) was prepared by mixing the wild-type plasmid (WT*28) and the wild-type plasmid (WT*6) so that the concentration of each plasmid becomes 400 copies/µL. Further, a plasmid sample (mt) was prepared by mixing the mutant-type plasmid (mt*28) and the mutant-type plasmid (mt*6) so that the concentration of each plasmid becomes 400 copies/µL. PCR and Tm analysis were performed in the same manner as the plasmid in Example B1 except that this plasmid sample and the PCR reaction solution shown in Table 11 were used.

### (Blood)

Blood was collected using a EDTA blood collection tube. It was preliminarily confirmed that the polymorphisms, UGT1A1*28 and UGT1A1*6, of this blood sample were both heterozygotes (wild/mutant). PCR and Tm analysis were performed in the same manner as the blood sample in Example B1 except that the blood collected as described above and the PCR reaction solution shown in Table 11 were used.

### (Control)

17 µL of the diluent 2 was treated at 95°C for 10 minutes and added to 46 µL of the PCR reaction solution, and then PCR and Tm analysis were performed in the same manner as the control in Example B1.

The Tm value of a double-stranded nucleic acid composed of the Tm control oligonucleotide and the Tm control probe is 57°C. Therefore, when the peak is detected at 57°C in the Tm analysis, it can be determined that a temperature control unit and a signal detecting unit of a Tm analyzing device function normally. The Tm values under the normal temperature condition are as follows:
(i) Tm value of a double-stranded nucleic acid composed of a Tm control oligonucleotide and a Tm control probe: 57°C
(ii) Tm value of a double-stranded nucleic acid composed of a wild-type UGT1A1*6 and a UGT1A1*6 probe: 47°C
(iii) Tm value of a double-stranded nucleic acid composed of a mutant-type UGT1A1*6 and a UGT1A1*6 probe: 55°C
(iv) Tm value of a double-stranded nucleic acid composed of a wild-type UGT1A1*28 and a UGT1A1*28 probe: 47°C
(v) Tm value of a double-stranded nucleic acid composed of a mutant-type UGT1A1*28 and a UGT1A1*28 probe: 52°C

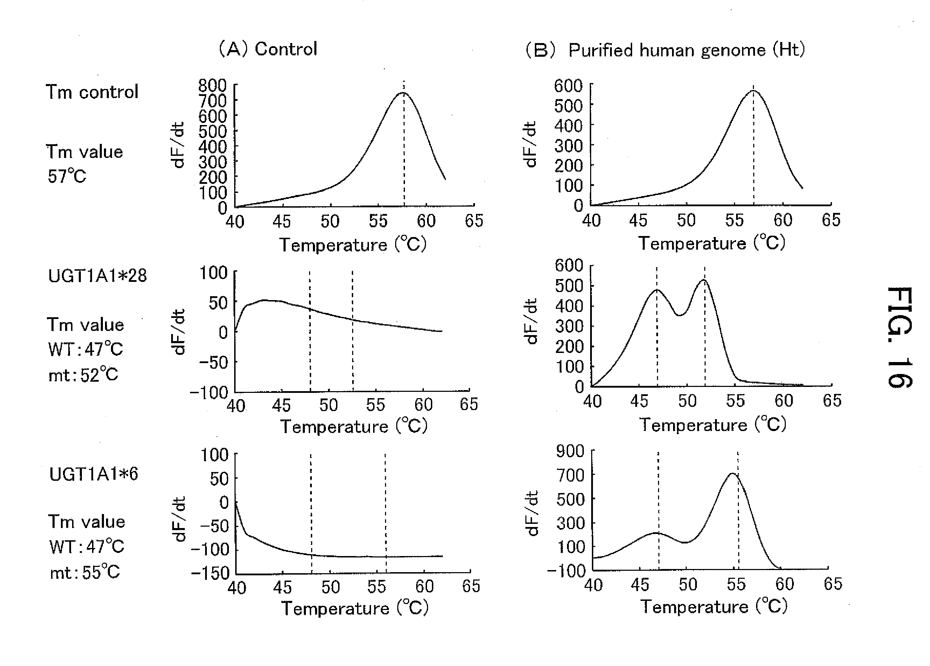

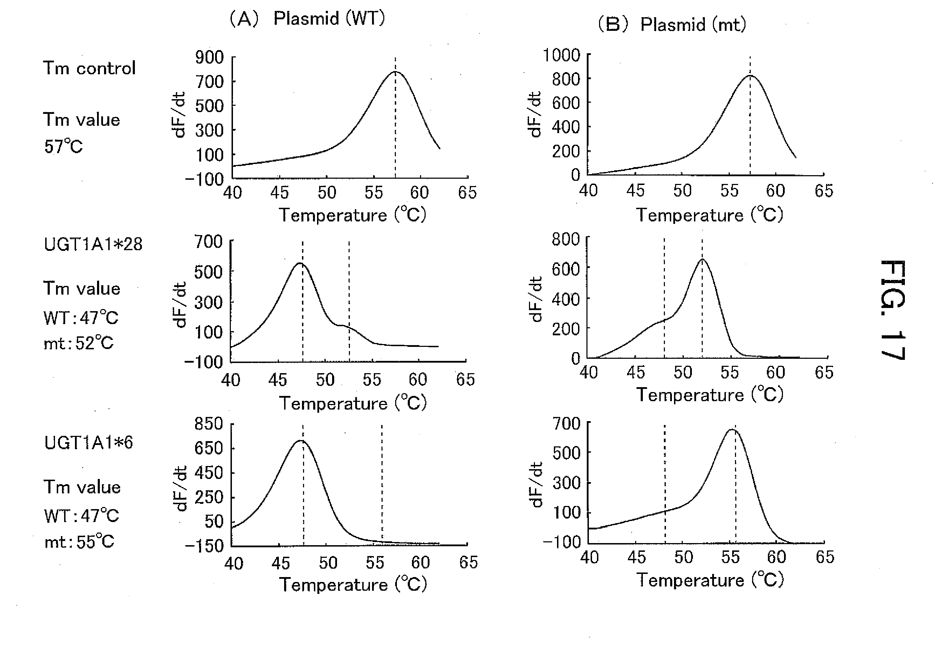

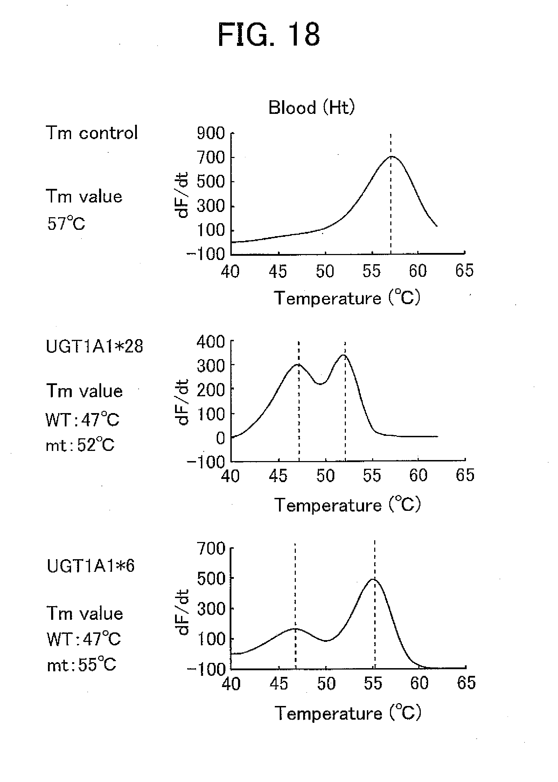

The results thereof are shown in FIGs. 16A to 18. FIGs. 16A to 18 show graphs of the Tm analysis showing the change in fluorescence intensity accompanying the temperature rise. In each of the graphs shown in FIGS. 16A to 18, the horizontal axis indicates temperature (°C) at the time of measurement. The vertical axis indicates the change in fluorescence intensity, and "d fluorescence intensity change amount / dt" (dF/dt), which is the differential value of the fluorescence intensity change amount is used as the unit. FIG. 16A shows the result of the control, FIG. 16B shows the result of the purified human genome, FIG. 17A shows the result of the wild-type plasmid (WT), FIG. 17B shows the result of the mutant-type plasmid (mt), and FIG. 18 shows the result of the blood. In each of FIGs. 16A to 18, the graph (Tm control) on the upper side shows the fluorescence change on the basis of the Tm control probe, the graph (UGT1A1*28) on the middle shows the fluorescence change on the basis of the UGT1A1*28 probe, and the graph (UGT1A1*6) on the lower side shows the fluorescence change on the basis of the UGT1A1*6 probe.

As shown in FIG. 16A, in the control, the peak was detected only at around 57°C with respect to the Tm control probe and the peak was not detected with respect to the UGT1A1*28 probe and the UGT1A1*6 probe. As shown in FIG. 16B, the peak was detected at around 57°C with respect to the Tm control probe, the peaks were detected at around 47°C and 52°C with respect to the UGT1A1*28 probe, and the peaks were detected at around 47°C and 55°C with respect to the UGT1A1*6 probe. As shown in FIG. 17A, the peak was detected at around 57°C with respect to the Tm control probe, the peak was detected at around 47°C with respect to the UGT1A1*28 probe, and the peak was detected at around 47°C with respect to the UGT1A1*6 probe. As shown in FIG. 17B, the peak was detected at around 57°C with respect to the Tm control probe, the peak was detected at around 52°C with respect to the UGT1A1*28 probe, and the peak was detected at around 55°C with respect to the UGT1A1*6 probe. As shown in FIG. 18, the peak was detected at around 57°C with respect to the Tm control probe, the peaks were detected at around 47°C and 52°C with respect to the UGT1A1*28 probe, and the peaks were detected at around 47°C and 55°C with respect to the UGT1A1*6 probe. Since the peak was detected at around 57°C with respect to the Tm control probe in all the reaction solutions, it was confirmed that the temperature control of the Tm analyzing device was carried out normally. Further, although PCR and Tm analysis were performed in the presence of the Tm control oligonucleotide and the Tm control probe, the peak was not detected at a temperature other than the aforementioned Tm values with respect to the UGT1A1*28 probe and the UGT1A1*6 probe. This tells that even when the Tm control oligonucleotide and the Tm control probe are used together, it does not affect the detection of an objective polymorphism. In this manner, the present invention allows the detection of the polymorphism while determining whether or not the temperature control and the signal detection of the Tm analyzing device are carried out normally. Therefore, further reliable polymorphism analysis can be performed.

### Industrial Applicability

In this manner, according to the present invention, a positive control and a negative control, which had to be detected separately, can be detected in one reaction system using the aforementioned control template. In this manner, by detecting the positive control and the negative control in one reaction system, for example, operations can be simplified and costs for a reagent and the like can be reduced.

## Claims

1. A control detection method for detecting controls showing
amplification efficiency of a reaction system when a target sequence of a specimen nucleic acid is amplified in the reaction system,
the controls being a positive control showing an occurrence of an amplification reaction in the reaction system and a negative control showing an occurrence of contamination of the reaction system with undesired nucleic acid,
the reaction system containing a primer, a detection probe, and a control template nucleic acid,
the primer being a primer capable of amplifying the target sequence,
the detection probe being a probe capable of hybridizing to the target sequence,
the control template nucleic acid capable of being amplified by the primer,
an amplification region of the control template nucleic acid amplified by the primer capable of being hybridized with the detection probe,
a Tm value (Tm_{C}) of a double-stranded nucleic acid composed of the amplification region of the control template nucleic acid and the detection probe being different from a Tm value (Tm_{A}) of a double-stranded nucleic acid composed of the target sequence and the detection probe,
the method comprising the following steps (a1) to (a3):
(a1) amplifying the control template nucleic acid in the reaction system using the primer;
(a2) performing a melting curve analysis in the presence of the detection probe using the reaction system obtained in the step (a1); and
(a3) determining whether the positive control and the negative control each are positive or negative on the basis of a peak in the melting curve, wherein the positive control and the negative control are detected in one reaction system.

2. The method according to claim 1, where in the step (a3), in the melting curve,
when a peak is present at the Tm value (Tm_{C}) of a double-stranded nucleic acid composed of the amplification region of the control template nucleic acid and the detection probe, the positive control is determined as positive;
when a peak is not present at the Tm value (Tm_{C}) of a double-stranded nucleic acid composed of the amplification region of the control template nucleic acid and the detection probe, the positive control is determined as negative;
when a peak is not present at a temperature other than the Tm value (Tm_{C}) of a double-stranded nucleic acid composed of the amplification region of the control template nucleic acid and the detection probe, the negative control is determined as negative; and
when a peak is present at a temperature other than the Tm value (Tm_{C}) of a double-stranded nucleic acid composed of the amplification region of the control template nucleic acid and the detection probe, the negative control is determined as positive.

3. The method according to claim 1, wherein a length of a hybridization region in the amplification region of the control template nucleic acid to which the detection probe can hybridize is different from that of a hybridization region in the target sequence to which the detection probe can hybridize.

4. The method according to claim 1, wherein the detection probe is a base sequence complementary to a predetermined region of the target sequence, and
the control template nucleic acid has, at the amplification region thereof, a base sequence to which the detection probe can partially hybridize.

5. The method according to claim 4, wherein the control template nucleic acid has, at the amplification region thereof, a base sequence in which at least one of a 5' side and a 3' side is different from that of a hybridization region in the target sequence to which the detection probe can hybridize.

6. The method according to claim 1, wherein the detection probe has a base sequence partially complementary to the target sequence, and
the control template nucleic acid has, at the amplification region thereof, a base sequence complementary to the detection probe.

7. The method according to claim 6, wherein the control template nucleic acid has, at the amplification region thereof, a base sequence that is identical to a hybridization region in the target sequence to which the detection probe can hybridize, and adjacent thereto, a base sequence that is different from a region adjacent to at least one of a 5' side and a 3' side of the hybridization region in the target sequence.

8. The method according to claim 1, wherein a homology between a base sequence of a hybridization region in the amplification region of the control template nucleic acid to which the detection probe can hybridize and a base sequence of a hybridization region in the target sequence to which the detection probe can hybridize is less than 100%.

9. The method according to claim 8, wherein a complementarity of the hybridization region in the control template nucleic acid to the detection probe is different from that of the hybridization region in the target sequence to the detection probe.

10. The method according to claim 1, wherein the target sequence contains a target site that indicates a polymorphism, and
the detection probe contains a base sequence complementary to a region containing the target site in the target sequence.

11. The method according to claim 10, wherein the detection probe has a base sequence complementary to a region containing a mutant-type target site in the target sequence or a base sequence complementary to a region containing a wild-type target site in the target sequence.

12. The method according to claim 1, wherein a difference between the Tm value (Tm_{C}) of a double-stranded nucleic acid composed of the amplification region of the control template nucleic acid and the detection probe and the Tm value (Tm_{A}) of a double-stranded nucleic acid composed of the target sequence and the detection probe is 1 to 50°C.

13. The method according to claim 1, wherein the detection probe is a labeled probe having a labeling substance.

14. The method according to claim 13, wherein the labeling substance is a fluorescent substance.

15. A nucleic acid amplification method for amplifying a target sequence of a specimen nucleic acid in a reaction system, comprising the following steps (A) and (B):
(A) detecting controls showing amplification efficiency with respect to a reaction system containing the control template nucleic acid, the primer, and the detection probe by the control detection method according to claim 1; and
(B) amplifying the target sequence of the specimen nucleic acid, comprising the following step (b1):
(b1) amplifying the target sequence of the specimen nucleic acid in a reaction system containing the specimen nucleic acid, the primer, and the detection probe using the primer.

16. The method according to claim 15, wherein the following step (a1) of the step (A) and the step (b1) of the step (B) are performed simultaneously:
(a1) amplifying the control template nucleic acid in the reaction system using the primer.

17. The method according to claim 15, wherein the step (B) further comprises the following steps (b2) and (b3):
(b2) performing a melting curve analysis in the presence of the detection probe using the reaction system obtained in the step (b1); and
(b3) determining whether or not the target sequence of the specimen nucleic acid is amplified on the basis of a peak in the melting curve.

18. The method according to claim 17, where in the step (b3),
when a peak is present at a Tm value (Tm_{A}) of a double-stranded nucleic acid composed of the target sequence and the detection probe, it is determined that the target sequence is amplified; and
when a peak is not present at the Tm value (Tm_{A}) of a double-stranded nucleic acid composed of the target sequence and the detection probe, it is determined that the target sequence is not amplified.

19. The method according to claim 17, wherein the following step (a2) of the step (A) and the step (b2) of the step (B) are performed simultaneously:
(a2) performing a melting curve analysis in the presence of the detection probe using the reaction system obtained in the step (a1).

20. The method according to claim 19, where in the following step (a3) of the step (A),
when a positive control is determined as positive and a negative control is determined as negative, the determination of the step (b3) is made:
(a3) determining whether the positive control and the negative control each are positive or negative on the basis of a peak in the melting curve.

21. A melting curve analysis method of a reaction system containing a specimen nucleic acid, comprising the following steps (A) and (C):
(A) detecting controls showing amplification efficiency with respect to a reaction system containing the control template nucleic acid, the primer, and the detection probe by the control detection method according to claim 1; and
(C) performing a melting curve analysis, comprising the following steps (c1) and (c2):
(c1) amplifying a target sequence of the specimen nucleic acid in a reaction system containing the specimen nucleic acid, the primer, and the detection probe using the primer; and
(c2) performing the melting curve analysis in the presence of the detection probe using the reaction system obtained in the step (c1).

22. The method according to claim 21, wherein
the target sequence of the specimen nucleic acid contains an objective target site that shows polymorphism,
the detection probe is a probe capable of hybridizing to a region containing the target site in the target sequence, and
the step (C) further comprises the following step (c3):
(c3) detecting the polymorphism on the basis of a peak in the melting curve.

23. The method according to claim 22, wherein
the detection probe contains a base sequence complementary to a region containing a mutant-type target site in the target sequence, and
in the step (c3),
when a peak is present at a Tm value (Tmₘ) of a double-stranded nucleic acid composed of a target sequence containing the mutant-type target site and the detection probe, it is determined that the target site of the specimen nucleic acid is a mutant-type, and
when a peak is present at a Tm value (Tmw) of a double-stranded nucleic acid composed of a target sequence containing a wild-type target site and the detection probe, it is determined that the target site of the specimen nucleic acid is a wild-type.

24. The method according to claim 22, wherein
the detection probe contains a base sequence complementary to a region containing a wild-type target site in the target sequence, and
in the step (c3),
when a peak is present at a Tm value (Tmw) of a double-stranded nucleic acid composed of a target sequence containing the wild-type target site and the detection probe, it is determined that the target site of the specimen nucleic acid is a wild-type, and
when a peak is present at a Tm value (Tmₘ) of a double-stranded nucleic acid composed of a target sequence containing a mutant-type target site and the detection probe, it is determined that the target site of the specimen nucleic acid is a mutant-type.

25. The method according to claim 21, wherein the following step (a1) of the step (A) and the step (c1) of the step (C) are performed simultaneously, and the following step (a2) of the step (A) and the step (c2) of the step (C) are performed simultaneously:
(a1) amplifying the control template nucleic acid in the reaction system using the primer, and
(a2) performing a melting curve analysis in the presence of the detection probe using the reaction system obtained in the step (a1).

26. The method according to claim 22, where in the following step (a3) of the step (A),
when a positive control is determined as positive and a negative control is determined as negative, the determination of the step (c3) is made:
(a3) determining whether the positive control and the negative control each are positive or negative on the basis of a peak in the melting curve.

27. A control detection reagent used for the method for detecting controls according to claim 1, comprising:
a control template nucleic acid, wherein
the control template nucleic acid can be amplified by a primer capable of amplifying an objective target sequence,
an amplification region of the control template nucleic acid amplified by the primer can be hybridized with a detection probe capable of hybridizing to the target sequence, and
a Tm value (Tm_{C}) of a double-stranded nucleic acid composed of the amplification region of the control template nucleic acid and the detection probe is different from a Tm value (Tm_{A}) of a double-stranded nucleic acid composed of the target sequence and the detection probe.

28. The control detection reagent according to claim 27 further comprising:
the primer: and
the detection probe.

29. An analysis reagent used for the melting curve analysis method according to claim 21, comprising:
the control detection reagent according to claim 27; and
an amplification reaction reagent for a specimen nucleic acid containing the primer and the detection probe.

30. A method for designing a control template nucleic acid for detecting controls showing amplification efficiency of a reaction system when a target sequence of a specimen nucleic acid is amplified,
the control template nucleic acid being a template nucleic acid capable of detecting both a positive control showing an occurrence of an amplification reaction in the reaction system and a negative control showing an occurrence of contamination of the reaction system with undesired nucleic acid, the method comprising a step of:
designing a base sequence of the control template nucleic acid such that the control template nucleic acid can be amplified by a primer capable of amplifying the target sequence,
an amplification region of the control template nucleic acid amplified by the primer can be hybridized with a detection probe capable of hybridizing to the target sequence, and
a Tm value (Tm_{C}) of a double-stranded nucleic acid composed of the amplification region and the detection probe is different from a Tm value (Tm_{A}) of a double-stranded nucleic acid composed of the target sequence and the detection probe.

31. The use of a control template nucleic acid for detecting controls
showing amplification efficiency of a reaction system when a target sequence of a specimen nucleic acid is amplified,
the control template nucleic acid consisting of a base sequence designed such that
the control template nucleic acid can be amplified by a primer capable of amplifying the target sequence,
an amplification region of the control template nucleic acid amplified by the primer can be hybridized with a detection probe capable of hybridizing to the target sequence, and
a Tm value (Tm_{C}) of a double-stranded nucleic acid composed of the amplification region and the detection probe is different from a Tm value (Tm_{A}) of a double-stranded nucleic acid composed of the target sequence and the detection probe, wherein
the control template nucleic acid is used as a control template nucleic acid for detecting, in one reaction system, a positive control showing an occurrence of an amplification reaction in a reaction system and a negative control showing an occurrence of contamination of a reaction system with undesired nucleic acid together.
